(19) Europäisches Patentamt
European Patent Office
Office européen des brevets

(11) **EP 2 846 531 A1**

(12) **EUROPEAN PATENT APPLICATION**
published in accordance with Art. 153(4) EPC

(43) Date of publication:
**11.03.2015 Bulletin 2015/11**

(21) Application number: **13785310.7**

(22) Date of filing: **30.04.2013**

(51) Int Cl.:
*H04N 5/225* (2006.01)  *G02B 7/28* (2006.01)
*G02B 7/30* (2006.01)  *G03B 35/20* (2006.01)
*G03B 37/00* (2006.01)  *H04N 5/369* (2011.01)
*H04N 13/02* (2006.01)

(86) International application number:
**PCT/JP2013/062672**

(87) International publication number:
**WO 2013/165006 (07.11.2013 Gazette 2013/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **01.05.2012 JP 2012104371**

(71) Applicant: **Central Engineering Co., Ltd.**
**Tokyo 1000005 (JP)**

(72) Inventors:
• **HASEGAWA, Takayoshi**
**Yokohama-shi**
**Kanagawa 222-0033 (JP)**

• **TANAKA, Katsuhisa**
**Yokohama-shi**
**Kanagawa 222-0033 (JP)**
• **TSUNODA, Makoto**
**Yokohama-shi**
**Kanagawa 222-0033 (JP)**

(74) Representative: **Vigand, Philippe et al**
**Novagraaf International SA**
**3 chemin de l'Echo**
**1213 Onex Geneva (CH)**

(54) **STEREO CAMERA AND STEREO CAMERA SYSTEM**

(57) The present invention provides a technique that is capable of improving the precision of a distance measurement made using a stereo camera, and is capable of reducing the size of a stereo camera device. The stereo camera is equipped with left and right imaging systems (1a, 1b) having left and right optical systems (2a, 2b) and left and right imaging elements (3a, 3b), and derives the distance to an imaging target on the basis of an image signal acquired by imaging the imaging target by means of the left and right imaging systems (1a, 1b). The light receiving surface of the left and right imaging elements (3a, 3b) in the left and right imaging systems (1a, 1b) is caused to bend in the direction of the optical axis so as to correct an aberration in the left and right optical systems (2a, 2b).

FIG. 2

**Description**

TECHNICAL FIELD

The present invention relates to a stereo camera which calculates a distance to a subject in a forward area or generates a stereoscopic image by processing parallaxes of a plurality of images acquired from a plurality of cameras.

BACKGROUND ART

In the related art, there has been known a stereo camera where optical axes of two cameras are arranged to be separated by a predetermined interval (base line length) to derive a distance to a subject in a forward area by processing a parallax of two images obtained from the two cameras. The stereo camera is mounted as a distance measurement device for measuring the distance to the subject on a vehicle or like and is used to a collision avoidance system issuing inter-car distance warning or obstacle warning.

In addition, in recent years, display apparatuses of displaying a stereoscopic image in a flat-screen TV have been widely spread. In addition, there have been known devices capable of capturing and recording a stereoscopic image in electronic information devices using electronic imaging elements such as a digital still camera or a video camera. In this case, two cameras using a CMOS image sensor or a CCD image sensor as a solid state imaging element are arranged at left and right symmetric positions, and image information obtained by imaging a subject by the cameras is recorded as stereoscopic image data in a recording medium.

In the above-described stereo camera, the parallax generated by simultaneous imaging of the left and right cameras is generated due to the arrangement of the left and right cameras which are arranged to be separated by a certain distance d in the horizontal direction. However, in the case of performing the imaging by using an imaging optical system in a telescopic system, in some cases, the overlap areas of the left and right captured images may become small, so that it may be difficult to obtain a sufficient parallax. In order to solve the problem, a stereo camera configured to control at least a pair of the imaging optical systems arranged at the left and right sides and an angle of convergence formed by optical axes of the pair of the imaging optical systems according to subject distance information is generally provided.

In many cases, as described above, in the stereo camera, the distance to the subject is measured or the stereoscopic image is generated by focusing light being emitted from the subject and passing through the two imaging optical systems on the imaging elements and by processing the image signals obtained from the imaging elements. However, in some cases, in an area (particularly, a peripheral portion) of the imaging element which is far from the optical axis of the imaging optical system, due to influence of distortion aberration of the imaging optical system or image plane aberration, a quality of the obtained image signal is deteriorated. As a result, in some cases, there are problems in that an accuracy of the distance measurement is deteriorated or the quality of the stereoscopic image is deteriorated.

In addition, generally, if the parallax occurring between the left and right cameras is configured to be large by lengthening the base line length, it is possible to improve the accuracy of the distance measurement. Therefore, in the case where there is a deterioration in accuracy of the distance measurement as described above, the base line length needs to be somewhat lengthened in order to compensate for the deterioration. However, the configuration may hinder device miniaturization. In addition, the imaging optical system itself needs to have a large size in order to suppress the distortion aberration or the image plane aberration of the imaging optical system to be small, and for this reason, in some cases, it may be difficult to miniaturize the device.

CITATION LIST

PATENT LITERATURE

    Patent Literature 1: JP 2012-53303 A
    Patent Literature 2: JP 2007-101662 A
    Patent Literature 3: JP 2005-278133 A
    Patent Literature 4: JP 2001-284564 A
    Patent Literature 5: JP 7-95623 A

SUMMARY OF INVENTION

TECHNICAL PROBLEM

The present invention is to provide a technique capable of improving an accuracy of distance measurement or a quality of a stereoscopic image by a stereo camera or capable of miniaturizing a device of a stereo camera.

SOLUTION TO PROBLEM

The present invention relates to a stereo camera which includes a plurality of imaging units, each of which is configured to include an optical system and an imaging element, wherein the distance to the subject is measured and the stereoscopic image is generated from the image signal of the subject acquired by the imaging unit. In addition, a light receiving surface of the imaging element in the imaging unit is curved in an optical axis direction so as to correct aberration of the optical system in the imaging unit.

More specifically, there is provided a stereo camera which includes a plurality of imaging units, each of which is configured to include an optical system and an imaging element on which light passing through the optical system is focused to derive a distance to a predetermined subject or to generate a stereoscopic image containing the subject based on an image signal acquired by imaging the subject by the imaging units, wherein a light receiving surface of the imaging element in at least a portion of the imaging units is curved in an optical axis direction so as to correct aberration of the optical system which forms an image of the subject on the imaging element.

Herein, as described above, in some cases, in the case where an image of the subject is formed on the imaging element by the optical system, a central portion of the light receiving surface of the imaging element which is close to the optical axis has no problem, but in a peripheral portion of the imaging element which is far from the optical axis, focus blur or image distortion occurs due to the aberration of the optical system. In the stereo camera, in some cases, due to the aberration, the accuracy of the distance measurement is deteriorated, or the quality of the stereoscopic image is deteriorated.

On the contrary, in the present invention, the light receiving surface of the imaging element generally configured with a planar surface is curved in the optical axis direction so as to correct the aberration of the optical system. Accordingly, in the entire surface of the light receiving surface of the imaging element, it is possible to suppress focus blur, image distortion, or the like caused by the aberration of the optical system. As a result, in the stereo camera, it is possible to suppress the deterioration in accuracy of the distance measurement or the deterioration in quality of the stereoscopic image. In addition, since the base line length can be shortened to miniaturize the optical system itself, it is possible to facilitate the device miniaturization.

In addition herein, the aberration of the optical system includes distortion aberration, spherical aberration, astigmatism, and all types of aberration which can be suppressed by curving the light receiving surface of the imaging element besides the image plane distortion where the image plane of the optical system is deviated from a planar surface.

In addition, in the present invention, the imaging units may be arranged so that optical axes intersect each other with a predetermined angle of convergence, and instead of changing the angle of convergence according to the subject, an area for acquisition of the image signal in the light receiving surface of the imaging element may be changed according to the subject.

There is a case where the optical axes of the imaging units in the stereo camera are arranged to intersect at an intersection angle referred to as an angle of convergence. However, the amplitude of the angle of convergence influences the distance measurement or the generation of the stereoscopic image. For example, a subject located at the intersection of the optical axes forming the angle of convergence becomes a visible subject which is located on a screen during reproduction of the generated stereoscopic image.

In the stereo camera of related art, the angle of convergence is changed by mechanically rotating each of the imaging units. In this case, a mechanism for rotating the imaging units is required, so that the device configuration of the stereo camera is complicated and the size thereof is increased. On the contrary, in the present invention, instead of changing the angle of convergence according to the subject, the area for acquisition of the image signal in the light receiving surface of the imaging element is changed according to the subject.

Namely, the subject at the intersection of the optical axes of the imaging units is imaged at the optical axes on the imaging elements of the imaging units. On the contrary, in the case where the angle of convergence is assumed to be changed, the image of the subject which is located at the point as a new intersection of the optical axes is formed on the area which is deviated from the optical axes of the imaging elements. By acquiring the image signal of the area which is deviated from the optical axes in the imaging units, it is possible to obtain the effect equivalent to the effect obtained by the changing of the angle of convergence without actually rotating the imaging units (hereinafter, the actual angle of convergence obtained by the control is referred to as an actual angle of convergence).

In addition, even in the case where the light receiving surfaces of the imaging elements of the imaging units are planar surfaces, the similar control can be performed. However, in this case, since the peripheral portions of the light receiving surfaces of the imaging elements are used, the control is performed under the influence of the aberration of the optical system, and thus, there is a problem in that the accuracy of the distance measurement or the quality of the stereoscopic image is deteriorated according to the actual angle of convergence. On the contrary, in the present invention, since the imaging elements are curved so as to correct the aberration of the optical system, it is possible to maintain the accuracy of the distance measurement or the quality of the stereoscopic image irrespective of the value of the actual angle of convergence.

In addition, in the present invention, at least a portion of the optical systems in the imaging units may be integrally formed. Namely, in the above-described invention, the actual angle of convergence is changed by changing the area for acquisition of the image signal in the light receiving surfaces of the imaging elements depending on the subject without rotating the imaging units. Therefore, there is no need to change a relative position between the optical systems of the imaging units. By using this point, in the present invention, at least a portion of the optical systems of the imaging units is integrally formed. Accordingly, it is possible to further simplify the device configuration, and it is possible to facilitate the cost reduction of the device.

In addition, in the present invention, the imaging elements on which light passing through the integrally-formed optical systems is focused may be integrally formed. By doing so, it is possible to further simplify the device configuration, and it is possible to facilitate the cost reduction of the device.

In addition, according to the present invention, there is provided a command signal discrimination device which discriminates a command signal input to a predetermined apparatus based on the image signal acquired in the stereo camera described above. The command signal discrimination device is a device which discriminates a content of a command according to the image acquired by the stereo camera and applies the command to a digital device or the like. For example, in a smart TV device, the command signal discrimination device is a device which images a motion of a viewer in front of the device by using the stereo camera and discriminates a content of a commend such as channel changing or the like according to the motion.

If the above-described stereo camera is applied to the command signal discrimination device, distance measurement and image generation are performed with a higher accuracy, so that it is possible to improve the accuracy of the command discrimination. In addition, since it is possible to more accurately limit a spatial range of the command discrimination, it is possible to suppress the problems in that the command discrimination is influenced by a motion of a person outside the range.

In addition, in the present invention, the stereo camera may further include a holding unit which holds the imaging units so that optical axes thereof do not intersect each other in front of the optical axes and angles of view of the optical systems of the imaging units overlap each other, and a distance measuring unit which detects the distance to the subject from the image signal of the subject imaged by the imaging units in the portion where the angles of view overlap.

In the present invention, the imaging units are held so that the optical axes do not intersect in the forward area, that is, so that the optical axes are parallel to each other or further separated from each other as it goes to the forward direction and so that the angles of views of the optical systems of the imaging units overlap each other. In addition, the distance to the subject is detected from the image signal of the subject in the portion where the angles of view of the optical system of the imaging units overlap each other.

Accordingly, while maintaining the surveillance function over the wider range covering with the angles of view of the imaging units, it is possible to perform the distance measurement or the generation of the stereoscopic image by using the stereo camera in the range where the angles of view overlap each other. Particularly, the surveillance function is maintained over a wider angle of view by using wide-angle lenses as the optical systems of the imaging units, so that it is possible to increase the range where the distance measurement or the generation of the stereoscopic image can be performed by using the stereo camera.

For example, each angle of view of the optical systems may be larger than 135 degrees. Accordingly, in the case where two imaging units are used, it is possible to allow a total of the angles of view to be 270 degrees. In addition, in this case, the holding unit may hold the two imaging units so that an angle between the optical axes is 90 degrees. Therefore, it is possible to secure 225 degrees or more as at least a total of the angles of view. In addition, it is possible to secure a range of 45 degrees or more as the range where the distance measurement or the generation of the stereoscopic image can be performed by using the stereo camera.

In addition, in the case where fisheye lenses having an angle of view of 180 degrees are used as the optical systems and the holding unit holds the two imaging units so that the angle between the optical axes is 90 degrees, it is possible to allow a total of the angles of view to be 270 degrees. In addition, it is possible to secure a range of 90 degrees as the range where the distance measurement or the generation of the stereoscopic image can be performed by using the stereo camera. Therefore, in the case where the stereo cameras according to the present invention are installed at the corner portions having 90 degrees, it is possible to perform surveillance of a total of 270 degrees of the periphery of the corner portion.

In addition, in the present invention, the holding unit may allow a size of an overlap portion of the angle of view to be changeable by changing a holding angle of the optical systems. Therefore, it is possible to adjust a total of the angles of view where the surveillance can be performed and the angle range where the distance measurement or the generation of the stereoscopic image can be performed by using the stereo camera according to the installation portion of the stereo camera.

In addition, according to the present invention, there is provided a stereo camera system which includes a plurality of the stereo cameras described above to acquire an image signal by imaging a predetermined subject by at least one of the stereo cameras and to derive a distance from a measurement reference object to the subject based on the image signal, wherein the stereo cameras are arranged directly to the measurement reference object so as to image an outside from the measurement reference object, and wherein the distance from the measurement reference object to the subject is derived by using the image signal acquired by two or more of the stereo cameras.

Accordingly, the stereo cameras are arranged directly to the measurement reference object, and the distance from the measurement reference object to the subject is measured by using two or more of the stereo cameras. Therefore, it is possible to perform the distance measurement by using the interval of the stereo cameras as the base line length for the measurement reference object without a change. Accordingly, the base line length can be configured to be large by setting the interval of the stereo cameras to be large, so that it is possible to improve the accuracy of the distance measurement performed by using the stereo cameras.

In this case, the measurement reference object may be a car, and the stereo cameras may be arranged at four corners of the car. In addition, the measurement reference object may be a house, and the stereo cameras may be arranged at convex-shaped corners of the house. Therefore, since the measurement reference object itself is large, it is possible to increase the distance between the stereo cameras, and it is possible to increase the base line length. As a result, it is possible to improve the accuracy of the distance measurement using two or more stereo cameras.

In addition, in the present invention, the stereo cameras may be arranged to the measurement reference object so that the distance measurement of a periphery of the measurement reference object in all directions can be performed by the stereo cameras. Therefore, it is possible to perform the measurement of the distance to the subject in the periphery of the measurement reference object in all directions by using the stereo cameras.

In addition, in the present invention, in the case where the distance between the measurement reference object and the subject is the predetermined distance or less, the distance from the measurement reference object to the subject may be derived by using one of the stereo cameras.

Namely, for example, at the start time of the measurement, the distance from the measurement reference object to the subject is measured by using the two or more stereo cameras, and in the case where the distance between the measurement reference object and the subject is the predetermined distance or less, or in the case where the distance becomes the predetermined distance or less, the distance measurement of the distance to the subject is continuously performed by using one of the stereo cameras.

Accordingly, in a normal state, the distance measurement over a wider range is continuously performed by using the two or more stereo cameras, and in the stage where the subject is somewhat close to the measurement reference object, it is possible to intensively perform the measurement of the distance to the subject by using one stereo camera. In addition, in the case where the distance measurement is performed by using the two or more stereo cameras, it may be considered that the distance measurement is performed by using a portion of the imaging units in the stereo cameras. In this case, the distance measurement can be performed over a wider range, but it may be difficult to secure the accuracy of the measurement due to the distortion or the movement of the base line length. Therefore, according to the present invention, in this case, in the stage where the subject is somewhat close to the measurement reference object, it is possible to perform the distance measurement with a higher accuracy by using one stereo camera.

In addition, according to the present invention, there is provided a stereo camera system which includes a plurality of the stereo cameras to acquire an image signal by imaging a predetermined subject by at least one of the stereo cameras and to derive a distance from a measurement reference object to the subject based on the image signal, wherein the stereo cameras are arranged directly to the measurement reference object so as to image an outside from the measurement reference object, and wherein the distance from the measurement reference object to the subject is derived by using the image signal acquired by two or more of the stereo cameras.

Also in this case, the measurement reference object may be a car, and the stereo cameras may be arranged at four corners of the car. In addition, the measurement reference object may be a house, and the stereo cameras may be arranged at convex-shaped corners of the house.

In addition, the stereo cameras may be arranged to the measurement reference object so that the distance measurement of the entire periphery of the measurement reference object can be performed by the imaging units.

In addition, in the case where the distance between the measurement reference object and the subject is the predetermined distance or less, the distance from the measurement reference object to the subject may be derived by using one of the stereo cameras.

In addition, the stereo camera may include two imaging units, each of which includes an optical system having an angle of view of 135 degrees or more and an imaging element on which light passing through the optical system is focused, and the stereo camera may further include a holding unit which holds the two imaging units so that an angle between the optical axes is 90 degrees.

Accordingly, it is possible to secure 225 degrees or more as the angle of view of at least one stereo camera, and it is possible to secure a range of 45 degrees or more as the range where the distance measurement or the generation of the stereoscopic image can be performed by using one stereo camera. Therefore, it is possible to perform the distance measurement in the entire periphery of the measurement reference object by using a smaller number of the stereo cameras. In addition, in the stage where the subject is somewhat close to the measurement reference object, it is possible to perform the measurement of the distance to the subject over a wider angle range by using one stereo camera.

In addition, as an approach for solving the above-described problems of the present invention, a combination may be used if possible.

EFFECTS OF INVENTION

According to the present invention, it is possible to improve an accuracy of distance measurement using a stereo camera or a quality of a stereoscopic image, and it is possible to miniaturize a device of a stereo camera.

BRIEF DESCRIPTION OF DRAWINGS

Fig. 1 is a diagram illustrating a schematic configuration of an imaging system of a stereo camera in the related art.

Fig. 2 is a diagram illustrating a schematic configuration of an imaging system of a stereo camera according to a first embodiment of the present invention.

Fig. 3 is a diagram illustrating a schematic configuration of the stereo camera according to the first embodiment of the present invention.

Fig. 4 is a diagram for explaining a principle of an imaging system of a stereo camera according to a second embodiment of the present invention.

Figs. 5(a) and 5(b) are diagrams illustrating a schematic configuration of an imaging system of a stereo camera according to a third embodiment of the present invention.

Fig. 6 is a diagram illustrating a schematic configuration of a smart TV system according to a fourth embodiment of the present invention.

Fig. 7 is a diagram for explaining a principle of distance measurement using a general stereo camera.

Fig. 8 is a diagram for explaining problems in case of using a wide-angle lens in a general stereo camera.

Fig. 9 is a diagram for explaining effects in case of applying the present invention to a stereo camera using a wide-angle lens.

Fig. 10 is a diagram for explaining viewpoint conversion in case of using a stereo camera having a non-parallel optical axis.

Figs. 11 (a) and 11 (b) are diagrams illustrating a schematic configuration of an imaging system of a stereo camera according to a fifth embodiment of the present invention.

Fig. 12 is a schematic diagram illustrating an aspect where the imaging system of the stereo camera according to the fifth embodiment of the present invention is incorporated into an endoscope.

Figs. 13(a) and 13(b) are diagrams illustrating an another aspect of a schematic configuration of the imaging system of the stereo camera according to the fifth embodiment of the present invention.

Fig. 14 is a diagram illustrating a schematic configuration of a stereo camera system according to a sixth embodiment of the present invention.

Fig. 15 is a diagram illustrating a schematic configuration of a second aspect of the stereo camera system according to the sixth embodiment of the present invention.

Fig. 16 is a diagram illustrating a schematic configuration of a stereo camera system according to a seventh embodiment of the present invention.

Figs. 17 (a) and 17 (b) are diagrams illustrating a schematic configuration of a stereo camera system according to an eighth embodiment of the present invention.

Fig. 18 is a diagram illustrating a schematic configuration of a stereo camera system according to a ninth embodiment of the present invention.

Figs. 19(a) and 19(b) are diagrams illustrating a schematic configuration of a stereo camera system according to a tenth embodiment of the present invention.

Fig. 20 is a diagram illustrating a schematic configuration of an endoscope system where an imaging system of a stereo camera according to an eleventh embodiment of the present invention is incorporated into a capsule-type endoscope.

Fig. 21 is a diagram illustrating a schematic configuration of a stereo camera system according to a twelfth embodiment of the present invention.

Figs. 22 (a) and 22 (b) are diagrams illustrating a schematic configuration of an imaging system of a stereo camera according to a thirteenth embodiment of the present invention.

DESCRIPTION OF EMBODIMENTS

Hereinafter, embodiments of the present invention will be described with reference to the drawings.

<First Embodiment>

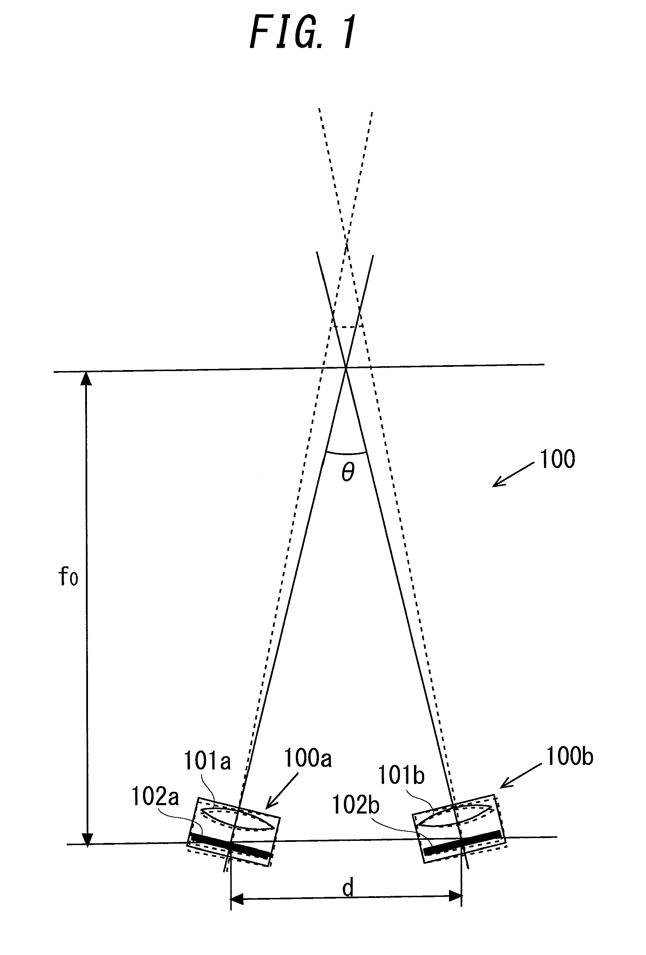

Fig. 1 illustrates a basic configuration of an imaging system 100 of a stereo camera in the related art. In Fig. 1, the stereo camera is a video camera or an electronic still camera which captures a subject image by using an optical system (lens system) and an imaging element and is configured to include a pair of left and right image systems of a left imaging system 100a at the left side and a right imaging system 100b at the right side. In the respective imaging systems, a movable left lens system 101a and a left imaging element 102a on which light passing through the left lens system 101a is focused are arranged along the respective optical axis, and a movable right lens system 101b and a right imaging element 102b on which light passing through the right lens system 101b is focused are arranged along the respective optical axis. In Fig. 1, although each of the left lens system 101a and the right lens system 101b is illustrated as a single lens, the number of lenses and the type of lenses are not particularly limited. In addition, in the figure, each of the left imaging element 102a and the right imaging element 102b may be a CCD sensor or a CMOS sensor.

In addition, in Fig. 1, the left imaging system 100a and the right imaging system 100b are arranged to be mutually inclined so that the optical axes intersect each other at a front position, that is, so that the left imaging system 100a and the right imaging system 100b has an angle of convergence θ. In addition, the left imaging system 100a and the right imaging system 100b are arranged with a spacing of a base line length d. $f_0$ is a distance to the subject. In addition, as indicated by broken lines in the figure, in the imaging system 100 of the stereo camera, the left imaging system 100a and the right imaging system 100b can be mechanically rotated. Accordingly, by mechanically rotating the left imaging system 100a and the right imaging system 100b, the angle of convergence θ therebetween can be changed.

It is possible to perform the measurement of the distance to the subject or to perform generation of a stereoscopic image by using the imaging system 100 of the stereo camera. For example, in the case of performing the measurement of the distance to the subject by using the imaging system 100 of the stereo camera, a displacement (parallax) of the subject imaged by the left imaging system 100a and the right imaging system 100b is measured by a stereo matching technique, and the distance is calculated on the basis of a well-known principle of triangulation. In addition, in the case of performing the generation of the stereoscopic image by using the imaging system 100 of the stereo camera, the angle of convergence θ is adjusted so that the optical axes of the left imaging system 100a and the right imaging system 100b intersect each other at a subject (hereinafter, referred to as a reference subject) which appears to exist on a screen during reproduction, and subjects are imaged by the left imaging system 100a and the right imaging system 100b.

When the images captured by the left imaging system 100a and the right imaging system 100b are viewed by the respective left and right eyes, according to the magnitude of the parallax obtained, a subject nearer than the reference subject appears to be protruded at the near side, and a subject farther than the reference subject appears to be recessed at the far side. By using this principle, it is possible to generate the stereoscopic image.

In the imaging system 100 of the stereo camera in the related art, as illustrated in Fig. 1, planar imaging elements has been used as the left imaging element 102a and the right imaging element 102b. In this case, although there is no problem in the vicinity of the optical axes of light receiving surfaces of the left and right imaging elements 102a and 102b, as it goes to the peripheral portions farther away from the optical axes on the light receiving surfaces, influence of aberration of the left and right lens systems 101a and 101b tends to increase. For example, if field curvature or distortion aberration is considered, the image planes of the left lens system 101a and the right lens system 101b are bent or distorted, so that, in some cases, focus blur or image distortion occurs as it goes farther away from the optical axes of the left lens system 101a and the right lens system 101b on the light receiving surfaces.

Therefore, in some cases, an accuracy of the distance measurement using the imaging system 100 of the stereo camera is deteriorated, or a quality of the stereoscopic image is deteriorated. Accordingly, in order to suppress the aberration of the left lens system 101a and the right lens system 101b, the respective lens systems need to be configured with a large size. In addition, with respect to the distance measurement, if the base line length d illustrated in Fig. 1 is set to be larger, the parallaxes of the images formed by the respective lenses become larger, and thus, the accuracy of the distance measurement may be improved. However, the size of the device also becomes large. Like this, for the reduction of the influence of the aberration of the left lens system 101a and the right lens system 101b in the imaging system 100 of the stereo camera in the related art, there occurs the problem in that the size of the device becomes large.

Therefore, in the embodiment, by curving the left imaging element 102a and the right imaging element 102b in order to correct the aberration of the left lens system 101a and the right lens system 101b, the influence of the aberration of the left lens system 101a and the right lens system 101b in the peripheral portions of the light receiving surfaces of the left imaging element 102a and the right imaging element 102b is suppressed.

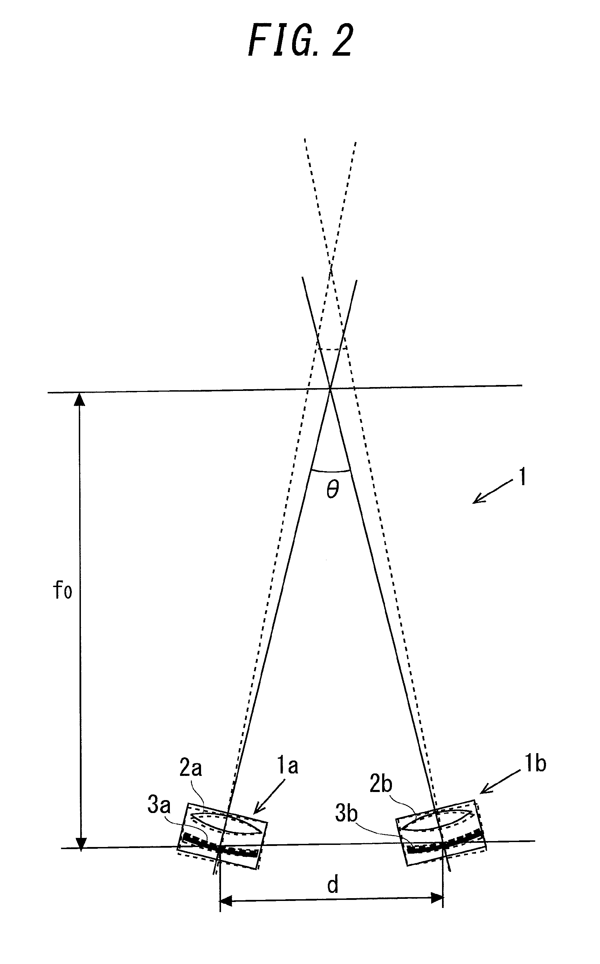

Fig. 2 illustrates an imaging system 1 of a stereo camera as an imaging unit according to the embodiment. In Fig. 2, the imaging system 1 of the stereo camera is configured to include a pair of left and right imaging systems of a left imaging system 1a at the left side of the figure and a right imaging system 1b at the right side. In the respective imaging systems, a movable left lens system 2a and a left imaging element 3a on which light passing through the left lens system 2a is focused are arranged along the respective optical axis, and a right lens system 2b and a right imaging element 3b on which light passing through the right lens system 2b is focused are arranged along the optical axes. The imaging system 1 is different from the imaging system 100 of the stereo camera in the related art illustrated in Fig. 1 in that the light receiving surfaces of the left imaging element 3a and the right imaging element 3b are formed with curved surfaces.

The shapes of the curved surfaces of the left imaging element 3a and the right imaging element 3b are defined so as to be coincident with the shapes of the image planes considering the aberration of the left lens system 2a and the right lens system 2b. Namely, the light passing through the left lens system 2a and the right lens system 2b is focused on the light receiving surfaces of the left imaging element 3a and the right imaging element 3b irrespective of whether or not to be in the vicinity of the optical axes or in the peripheral portions of the light receiving surfaces.

The left imaging element 3a and the right imaging element 3b which are formed with curved surfaces may be manufactured, for example, by adhering imaging element chips which is thinned by grinding on a substrate made of ceramic or the like so that the shapes of the surfaces are coincident with the shapes of the image planes of the left lens system 2a and the right lens system 2b. Otherwise, position regulating columns which regulates the positions in the thickness direction of imaging element chips may be prepared at a plurality of locations on a substrate made of ceramic or the like, the imaging element chips may be adhesively fixed to the position regulating columns in the state of being in contact with each other. Otherwise, portions of fixing peripheral portions and central portions of image elements chips in a substrate may be formed with separate members, and relative potions in the optical axis direction of the members of fixing the peripheral portion and the central portions may be allowed to be changed, so that the shapes of the curved surfaces of the imaging element chips can be adjusted. Besides, the method of manufacturing the left imaging element 3a and the right imaging element 3b formed with curved surfaces are not particularly limited.

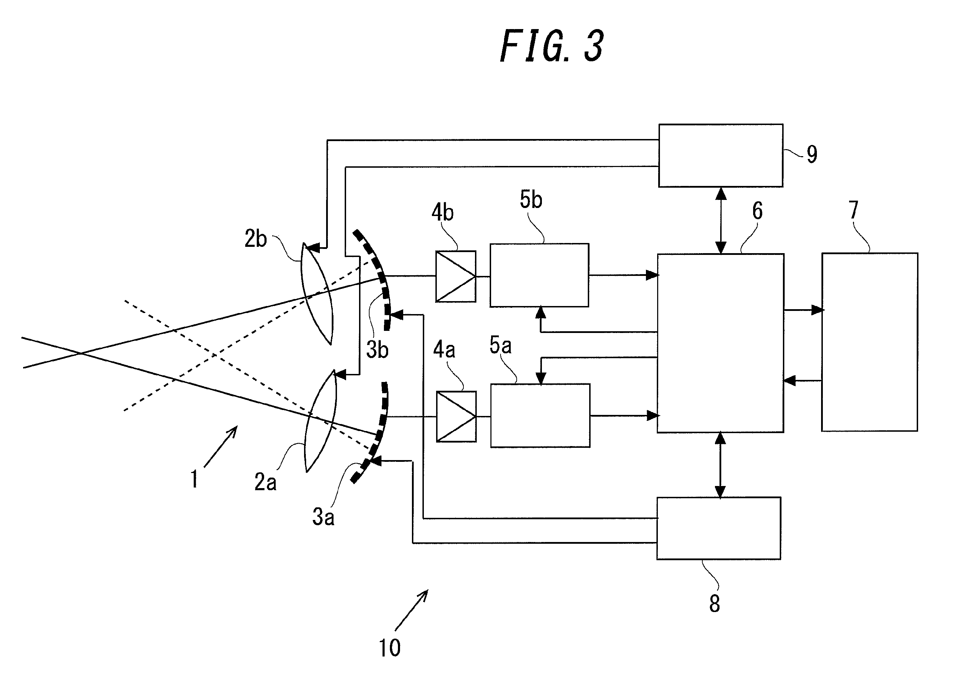

Fig. 3 illustrates a schematic configuration of a stereo camera 10 according to the embodiment. Image signals obtained by the left imaging element 3a and the right imaging element 3b of the imaging system 1 of the stereo camera are subjected to noise removal and amplification in a left amplification unit 4a and a right amplification unit 4b, respectively. Next, analog image signals output from the left amplification unit 4a and the right amplification unit 4b are subjected to AD conversion by the left AD converter 5a and the right AD converter 5b, respectively, and the resulting signals are input to a signal processing unit 6.

The signal processing unit 6 generates a driving signal for a sensor driving control unit 8 for driving the left imaging element 3a and the right imaging element 3b or a driving signal for a lens driving control unit 9 of moving the left lens system 2a and the right lens system 2b in the optical axis direction. In addition, in the case of the distance measurement, the distance to the subject is calculated from digital signals output from the left AD converter 5a and the right AD converter 5b, and in the case of the generation of the stereoscopic image, a signal process for generating left-eye images and right-eye images from the digital signals is performed. Therefore, in an image processing unit 7, in the case of the distance measurement, an image of display of measured distance is generated, and in the case of the generation of the stereoscopic image, an image process for generating the left-eye images and right-eye images are performed.

Heretofore, as described, in the imaging system of the stereo camera according to the embodiment, by forming each imaging element with curved surfaces so as to correct the aberration of each lens system, it is possible to acquire image signals based on high quality images without focus blur or distortion even in the peripheral portion of the imaging element. Therefore, it is possible to improve the accuracy of the distance measurement in the distance measurement using the stereo camera, and it is possible to generate a stereoscopic image having a higher quality.

In addition, since it is possible to perform the distance measurement with a high accuracy even on the subject which is imaged in the peripheral portions of the imaging elements, there is no need to lengthen the base line length to be so long, and the necessity of performing correction of aberration in the lens system itself is decreased. As a result, it is possible to facilitate miniaturization of the stereo camera.

<Second Embodiment>

In the first embodiment, the example of mechanically adjusting the angle of convergence θ formed by the optical axes of the left imaging system 1a and the right imaging system 1b similarly to the related art is described. However, in this embodiment, an example of changing image signal acquisition areas in the light receiving surfaces of the left imaging element 3a and the right imaging element 3b so as to obtain an effect electrically equivalent to the changing of the angle of convergence θ is described.

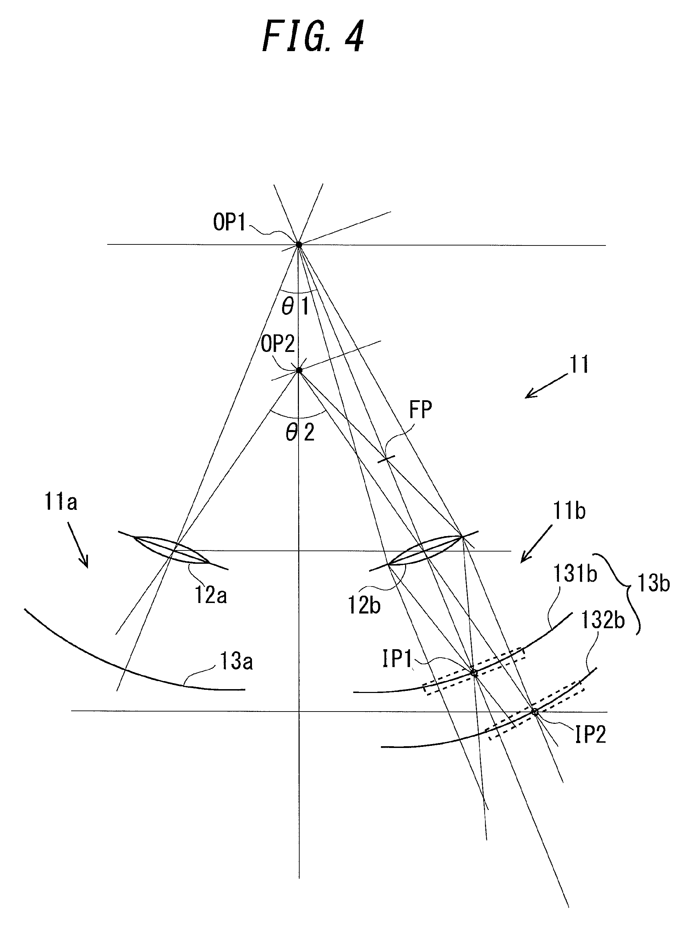

Fig. 4 illustrates an imaging system 11 of a stereo camera according to the embodiment. Similarly to the first embodiment, the imaging system 11 of the stereo camera is configured to include a left imaging system 11a and a right imaging system 11b, the left imaging system 11a is configured to include a left lens system 12a and a left imaging element 13a, and the right imaging system 11b is configured to include a right lens system 12b and a right imaging element 13b.

Fig. 4 illustrates imaging status on the right imaging element 13b by the right lens system 12b in the case of selecting a relatively far object point OP1 as a reference subject and the case of selecting a relatively near object point OP2 as a reference subject. The object point OP1 exists at the intersection of the optical axes of the left imaging system 11a and the right imaging system 11b, and in this case, the angle of convergence becomes an angle of convergence θ1. In the related art, in the case where the angle of convergence is changed from this state to θ2 and the reference subject is changed from OP1 to OP2, the left imaging system 11a and the right imaging system 11b are mechanically rotated so that the intersections of the optical axes become close to each other, and thus, the intersections of the optical axes are coincident with the object point OP2. In this case, since a driving source such as a motor for rotating the left imaging system 11a and the right imaging system 11b and a movement transmission mechanism are required, the imaging system 11 of the stereo camera becomes complicated and large, and the cost thereof is increased.

On the contrary, in the embodiment, by changing the image signal acquisition area in the light receiving surface of each imaging element, it is possible to substantially change the angle of convergence without mechanically rotating the left imaging system 11a and the right imaging system 11b. Herein, in Fig. 4, a right imaging element 131b denotes a right imaging element in the case of selecting the object point OP1 as a reference subject, and a right imaging element 132b denotes a right imaging element in the case of selecting the object point OP2 as a reference subject. Both are the same imaging element.

In fact, in the case of selecting the object point OP1 as a reference subject and in the case of selecting the object point OP2 as a reference subject, the position in the optical axis direction of the right lens system 12b can be changed by the lens driving control unit 9, and thus, in both of the cases, image points are formed on the right imaging element 13b at the same position. For simplifying Fig. 4, the right lens system 12b is illustrated to be fixed, and the right imaging element 13b is illustrated to be movable.

In Fig. 4, in the case of selecting the object point OP1 as a reference subject, light emitted from the object point OP1 passes through the lens system 12b and is focused on the intersection to the optical axis of the right imaging element 131b, so that an image point IP1 is formed. Therefore, in the case of selecting the object point OP1 as a reference subject, the distance measurement or the generation of the stereoscopic image is performed according to image information of, for example, areas surrounded by broken lines in the central portion of the right imaging element 131b.

Next, in the case of selecting the object point OP2 as a reference subject, the light emitted from the object point OP2 passes through the lens system 12b and is focused on a point other than the intersection to the optical axis of the right imaging element 132b, so that an image point IP2 is formed. At this time, as seen from Fig. 4, in comparison to the case of selecting the object point OP1 as a reference subject, the position of the image point in the right imaging element 13b is relatively changed. In this case, the distance measurement or the generation of the stereoscopic image is performed by using the image information of the area surrounded by the broken line around the image point IP2. Accordingly, it is possible to obtain the effect equivalent to that of the case of mechanically rotating the left imaging system 11a and the right imaging system 11b.

As described above, in the embodiment, the configuration of the imaging system 11 of the stereo camera can be simplified and miniaturized, so that it is possible to further facilitate cost reduction.

In addition, the electrical changing of the angle of convergence can be also performed by the imaging system 100 of the stereo camera in the related art as illustrated in Fig. 1. However, in the case where the electrical changing of the angle of convergence is performed by the imaging system 100 of the stereo camera in the related art, since the left imaging element 102a and the right imaging element 102b are configured with planar surfaces, at the IP2 or furthermore at the peripheral portion side, due to the influence of the aberration of the left lens system 101a and the right lens system 101b, the accuracy of the distance measurement or the quality of the stereoscopic image are lowered as a whole. On the contrary, in the embodiment, since the right imaging element 13a and the left imaging element 13b are configured with curved surfaces so as to correct the aberration of the left lens system 12a and the right lens system 12b, even though the angle of convergence is electrically changed, it is possible to maintain the accuracy of the distance measurement or the quality of the stereoscopic image to be high.

<Third Embodiment>

Next, a third embodiment of the present invention will be described. As described in the second embodiment, in the case where the electrical changing of the angle of convergence is employed in the present invention, there is no need to mechanically rotate the left imaging system and the right imaging system. In addition, in the case where wide-angle lenses (or pan-focus lenses) having a deep depth of field are used for the left and right lens systems, the movement in the optical axis direction for focus adjustment of the lens systems may be omitted. In this case, all of the left and right lens systems and the left and right imaging elements may be integrated. In the embodiment, such an integrated imaging system of a stereo camera will be described.

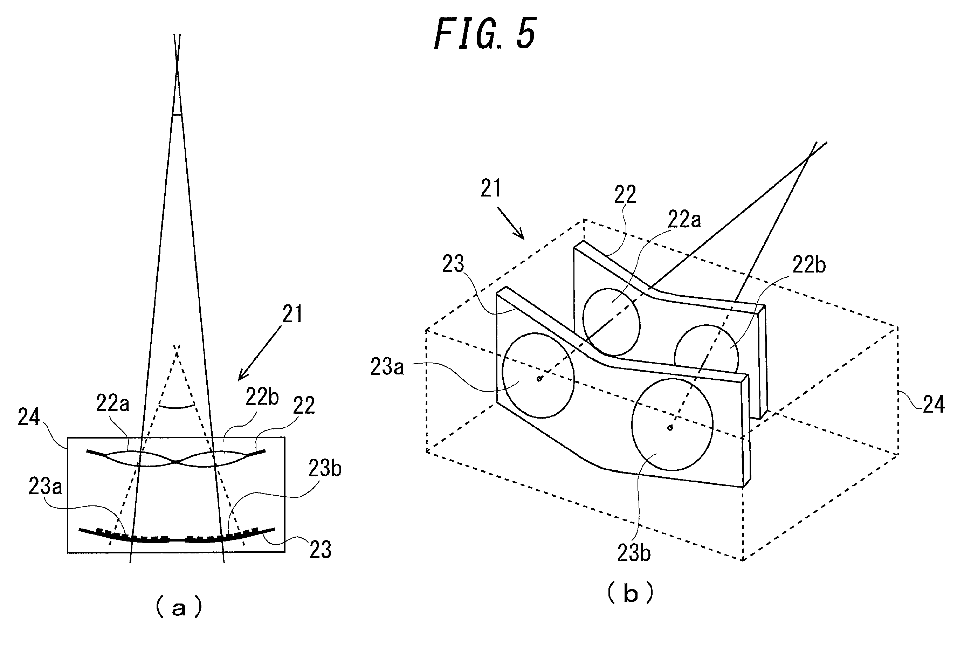

Figs. 5(a) and 5(b) illustrate an integrated imaging system 21 according to the embodiment. Fig. 5(a) illustrates a plan diagram of the integrated imaging system 21, and Fig. 5(b) illustrates a perspective diagram thereof. In Figs. 5(a) and 5(b), a left lens system 22a and a right lens system 22b are integrally formed to constitute an integrated lens system 22. The integrated lens system 22 may be formed by integrally molding the left lens system 22a and the right lens system 22b by using a resin or a glass material or may be formed by separately forming the left lens system 22a and the right lens system 22b and fixing to a holder by using a method such as adhesion.

In addition, similarly, in the embodiment, an integrated imaging element 23 is formed by integrally forming a left imaging element 23a and a right imaging element 23b. Furthermore, the integrated lens system 22 and the integrated imaging element 23 are fixed to an imaging system frame 24. In this manner, the imaging system 21 of the stereo camera according to the embodiment has a configuration where all the left and right lens systems and the left and right imaging elements are fixed and are not required to move. Therefore, due to the simplification of the structure of the stereo camera, it is possible to improve the reliability and to facilitate the cost reduction of the device. In addition, it is possible to improve the accuracy of the relative positions of the left and right lens systems and the left and right imaging elements.

In addition, in the embodiment, for example, the direction of the light passing through the left lens system 22a may be changed by a mirror, and the light may be focused on the light receiving surface of the right imaging element 23b. Therefore, the image information according to the light passing through the left lens system 22a and the right lens system 22b can be acquired by one image element, so that it possible to facilitate the simplification, miniaturization, and cost reduction of the device.

In addition, in the embodiment, the example where the wide-angle lenses (or pan-focus lenses) having a deep depth of field are used for the left and right lens systems and the relative movement between the left and right lens systems and the left and right imaging elements for focus adjustment is omitted is described. However, the wide-angle lenses (or pan-focus lenses) of which focus adjustment is not necessary may not be used in the left and right lens systems, and the relative movement between the left and right lens systems and the left and right imaging elements for focus adjustment may be performed by the lens driving control unit 9 illustrated in Fig. 3. Even in this case, since the adjustment of the position of only the integrated lens system 22 by the lens driving control unit 9 is sufficiently good, it is possible to simplify the mechanism and the control in comparison to the case of independently adjusting the positions of the left lens system 22a and the right lens system 22b.

<Fourth Embodiment>

Next, a fourth embodiment of the present invention will be described. In the fourth embodiment, an example where the stereo camera according to the present invention is used as an information input device of a smart TV will be described. Herein, the smart TV denotes a television set which have an access to the Internet in addition to television broadcast to enable an interactive using method. In addition, in the smart TV, the information input performed by using motion of a person (user) is considered.

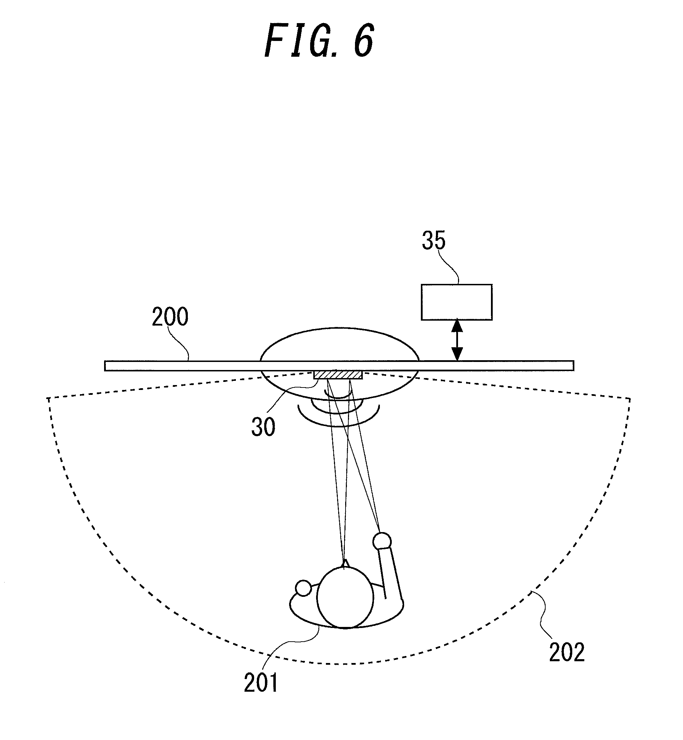

Fig. 6 is a plan diagram illustrating a usage state of a smart TV system 200 according to the embodiment. In the smart TV system 200, as viewed from the upper portion, a stereo camera 30 is installed at the center. In addition, a calculation device 35 which the image information acquired by the stereo camera 30 is transmitted to and which determine a content of the input from the image information is installed. The stereo camera 30 according to the embodiment is the same as the stereo camera described in the first embodiment in that the stereo camera is configured to include the left and right lens systems (not illustrated) and the left and right imaging elements formed with curved surfaces. Therefore, the accuracy of the distance measurement is high, and it is possible to recognize the reaction of a user 201 with a good accuracy. In addition, the influence of the aberration of the left and right lens systems even in the peripheral portions of the light receiving surfaces of the left and right imaging elements does not easily appear, and it is possible to perform the distance measurement with a high accuracy.

As a result, as indicated by 202 in the figure, it is possible to accurately perform recognition of the reaction of the user in a wider space. In addition, since the location where the user recognized as an information source is located can be more accurately set, even in the case where plural users are located in the room, it is possible to more accurately recognize the users as information sources. As a result, for example, it is possible to suppress the problem in that the operations of the smart TV system 200 are influenced by the reaction of users other than the information sources. In addition, in the smart TV system 200 according to the embodiment, the command signal discrimination device is configured to include the stereo camera 30 and the calculation device 35.

<Fifth Embodiment>

Next, a fifth embodiment of the present invention will be described. In the embodiment, an example where the present invention is applied to a stereo camera where fisheye lenses having an angle of view of 180 degrees are used as the lenses of the left and right lens systems and the two fisheye lenses are fixed so that the optical axes form an angle of 90 degrees will be described.

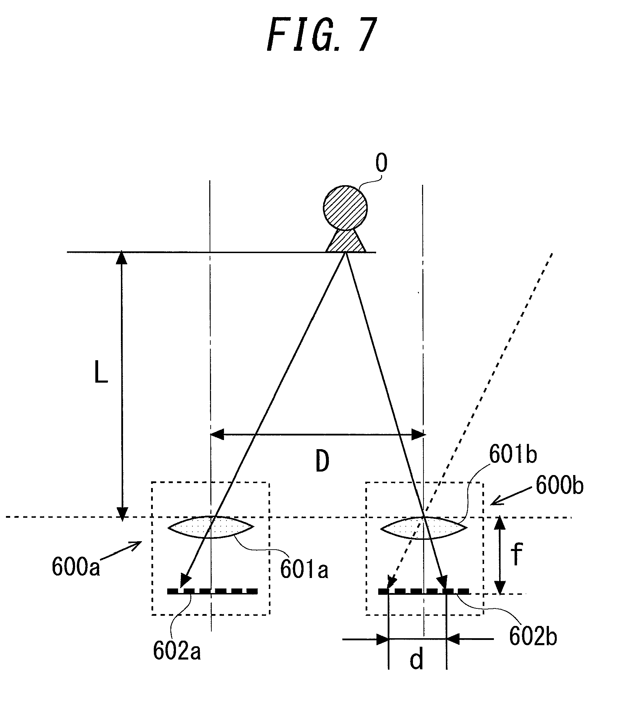

Herein, the principle of measurement of the distance to the object by the stereo camera according to the embodiment will be described with reference to Figs. 7 to 10. Fig. 7 illustrates a case of a stereo camera for distance measurement including a left imaging system 600a and a right imaging system 600b as two imaging systems having general planar-shaped imaging elements. The stereo camera compares images obtained from the two imaging systems of the left imaging system 600a and the right imaging system 600b to acquire distance measurement information. Therefore, similarity of the duplicate images obtained by the two imaging systems becomes important.

In the case where the angles of view of the left lens system 601a and the right lens system 601b of the two imaging systems are relatively narrow angles, the image distortion of each lens system is small. However, in the case where the angles of view of the left lens system 601a and the right lens system 601b are wide angles, in the peripheral portions far from the central portion (centers of the optical axes) in a left imaging element 602a and a right imaging element 602b, the image distortion caused by optical aberration becomes large. For this reason, in some cases, similarity of the images obtained by the two imaging systems is deteriorated, and thus, problems of the deterioration in accuracy of the distance measurement or the reduction of the measurable distance may occur.

As illustrated in Fig. 7, in many cases, in the stereo camera for distance measurement including the two imaging system having general planar-shaped imaging elements, the left imaging system 600a and the right imaging system 600b are arranged so that the optical axes thereof are parallel to each other. Therefore, light incident from the object O as a distance measurement target on the left imaging system 600a is focused on the planar-shaped imaging element 602a though left lens system 601a. Similarly, light incident from the object O on the right imaging system 600b is focused on the planar-shaped imaging element 602b through the right lens system 601b. If the right imaging system 600b is considered, a difference between the position of the image formed on the planar-shaped imaging element 602b and the position of the image in the case where light parallel to the light incident on the left imaging system 600a is assumed to be formed on the imaging element 602b corresponds to a parallax.

If a distance between the optical axes of the left lens system 601a and the right lens system 601b of the two imaging systems is denoted by a base line length D, if focal lengths of the left lens system 601a and the right lens system 601b are denoted by f, and if a total parallax is denoted by d, a distance L from the left lens system 601a and the right lens system 601b to the object O is calculated according to Mathematical Formula (1).

[Mathematical Formula 1]

$$L = D \cdot f / d \quad \ldots\ldots \quad (1)$$

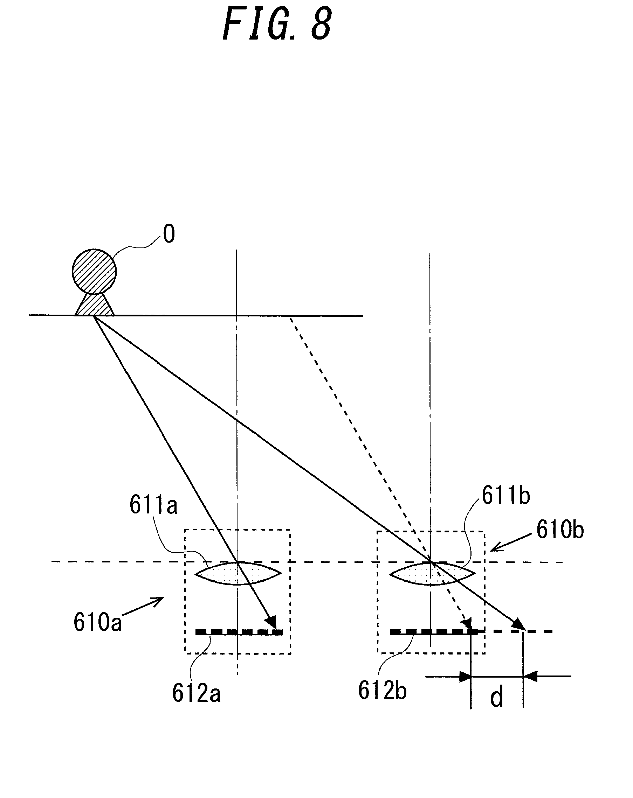

Next, an example where wide-angle lenses are used as a left lens system 611a and a right lens system 611b in a left imaging system 610a and a right imaging system 610b of a similar stereo camera will be considered. In this case, as illustrated in Fig. 8, light from the object O which is far from, particularly, the centers of the optical axes is focused at a position which is far from the central portions (centers of optical axes) of a planar-shaped left imaging element 612a and a planar-shaped right imaging element 612b. In fact, information existing within the angles of views of the left lens system 611a and the right lens system 611b is imaged on the left imaging element 612a and the right imaging element 612b. However, since broadened information is concentrated on a small number of pixels on the left imaging element 612a and the right imaging element 612b as it is far from the centers of the optical axes, there is a problem in that resolution is greatly deteriorated. In addition, at this time, as it goes to the ends of the left imaging element 612a and the right imaging element 612b, the image of the object O is greatly distorted. Therefore, there is a problem in that the similarity of the images required for parallax calculation is also greatly deteriorated. In addition, this tendency becomes greatly large at the opposite side of the object O, for example, the right imaging element 612b in Fig. 8.

In general, as a method of calculating the parallax from the images of the two imaging systems, a method called block matching is used. In this method, in order to evaluation the similarity between two images, by cutting a predetermined area from compared image and comparing positions of the cut area in the imaging elements, the displacement of the image, that is, the parallax is obtained. For this reason, if the resolution of the one-side imaging system is deteriorated or the image distortion in increased, there is a problem in that the displacement of the image cannot be calculated, the accuracy of the distance measurement of the distance to the object is deteriorated, or the distance measurement cannot be performed.

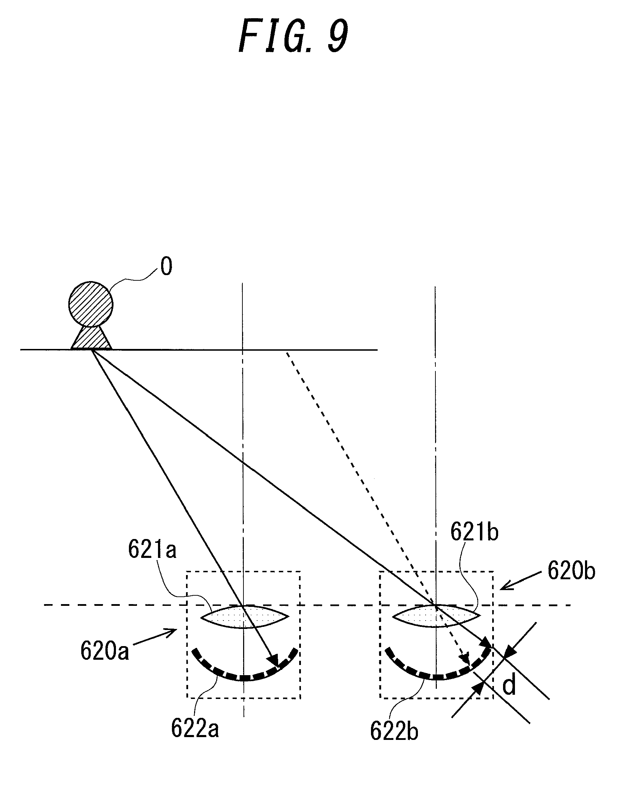

Next, an example where a curved sensor according to the present invention is applied to an imaging system having a wide-angle lens system will be described with reference to Fig. 9. Herein, instead of the planar-shaped left imaging element 612a and the planar-shaped right imaging element 612b illustrated in Fig. 8, a curved left imaging element 622a and a curved right imaging element 622b are arranged so as to correct the optical aberration of areas which are far from optical axes of a left lens system 621a and a right lens system 621b. Therefore, even in the case where the light emitted from the object O is focused on the locations which are far from the centers of the left and right imaging elements 622a and 622b, since the optical distortion can be solved, it is possible to obtain the accuracy of the distance measurement equivalent to that of the stereo camera implemented with a narrow angle.

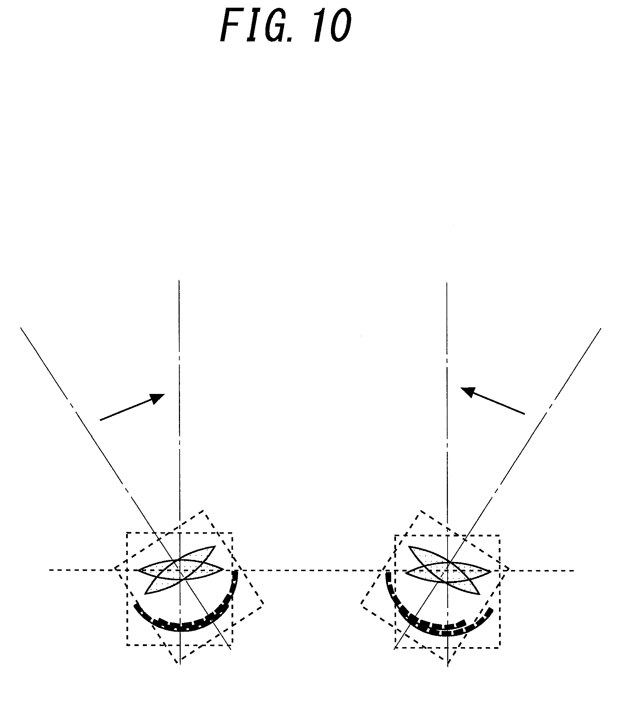

In addition, in the stereo camera, by using the wide-angle lenses described above as the left and right lens systems and arranging the optical axes of the left and right imaging systems not to be parallel to each other but to have an angle which is far from each other as it goes forward, so that it is considered that it is possible to obtain a very large field of view. In the case where the stereo camera having such non-parallel optical axes is used, as illustrated in Fig. 10, the images obtained by the left and right image elements are viewpoint-converted so as to correspond to the case where the optical axes of the left and right imaging systems are parallel to each other, and the parallax can be obtained from the overlap portion of the images by the left and right image systems after the viewpoint conversion. In addition, in many cases, the overlap portion of the images in the stereo camera having such non-parallel optical axes becomes the area which is far from the optical axes. Therefore, by using the curved imaging elements in order to correct the optical aberration of the areas which are far from the optical axes as the left and right imaging elements of the stereo camera, it is possible to obtain the effect of solving the optical distortion and the effect of improvement of the accuracy of the distance measurement equivalent to or more than the effects of the case where the optical axes of the left and right imaging systems are parallel to each other.

By applying the curved sensors to the stereo camera, it is possible to simplify the data process for the viewpoint conversion and the correction of the image distortion, and it is possible to reduce the load to the information processing device (CPU). In the embodiment, by using the above-described characteristics, fisheye lens having an angle of view of 180 degrees (or about 180 degrees) are used as the left and right lens systems, and the left and right imaging systems are fixed so that the optical axes have an angle of 90 degrees. Hereinafter, the embodiment will be described more in detail.

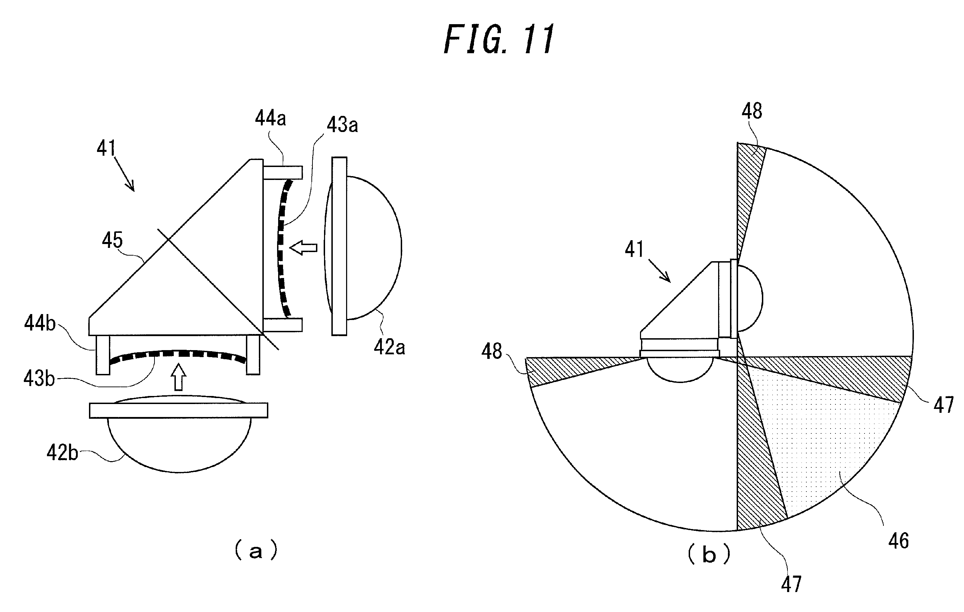

Figs. 11(a) and 11(b) illustrate a schematic configuration of an imaging system 41 of the stereo camera according to the embodiment. Fig. 11(a) is a diagram of a schematic configuration of the imaging system 41 of the stereo camera as viewed from the upper portion, and Fig. 11 (b) is a diagram for explaining usage states.

The imaging system 41 of the stereo camera according to the embodiment is configured to include ultra-wide-angle lenses (fisheye lenses) having an angle of view of 180 degrees as a left lens system 42a and a right lens system 42b. In addition, a left holder 44a and a right holder 44b for holding the left lens system 42a and the right lens system 42b by using peripheral portions (ribs) of the lenses are fixed so as to have an angle of 90 degrees with respect to a lens holding unit 45. In addition, a left imaging element 43a and a right imaging element 43b which are formed with curved surfaces are installed inside the left holder 44a and the right holder 44b so as to correct the aberration of the left lens system 42a and the right lens system 42b.

In the imaging system 41 of the stereo camera, since the left lens system 42a and the right lens system 42b having an angle of view of 180 degrees are held so that the optical axes have an angle of 90 degrees, as illustrated in Fig. 11(b), the entire imaging system 41 of the stereo camera can have a field of view of 270 degrees. In addition, it is possible to measure the distance to the subject from the image information obtained in the area 46 (area 46 also includes an area 47) where the angles of view of the left lens system 42a and the right lens system 42b overlaps each other in Fig. 11(b).

In addition, in the embodiment, since the left imaging element 43a and the right imaging element 43b formed with curved surfaces are installed so as to correct the aberration of the left lens system 42a and the right lens system 42b, it is possible to reduce the influence of the aberration in the peripheral portions of the light receiving surfaces of the left imaging element 43a and the right imaging element 43b. Therefore, for example, it is possible to improve the quality of the images in areas 47 and 48 of Fig. 11 (b), and it is possible to improve the accuracy of the distance measurement in the areas 47 and 48.

In addition, when the imaging system 41 of the stereo camera according to the embodiment is installed, the imaging system 41 may be installed in the center having, for example, an angle of 90 degrees. Accordingly, it is possible to use the stereo camera as a surveillance camera for all directions around the corner. In addition, it is possible to perform the distance measurement of the distance to the subject at an angle of 90 degrees among them.

In addition, in the embodiment, the holding unit 45 may be configured so that the angles between the left lens system 42a and the left imaging element 43a and the angles between the right lens system 42b and the right imaging element 43b are changeable. In this case, it is possible to adjust a surveillance angle and a distance measureable angle according to the installation sites or purposes of the imaging system 41 of the stereo camera.

In addition, in the embodiment, the example where the fisheye lenses having an angle of view of 180 degrees are used as the left lens system 42a and the right lens system 42b is described. However, the angles of view of the left lens system 42a and the right lens system 42b are not limited to 180 degrees. If each angle of view is larger than 135 degrees, it is possible to secure a field of view of 270 degrees as the imaging system 41 of the stereo camera, and it is possible to secure the portion of which angles of view overlap (distance measureable portion). In addition, fisheye lenses having an angle of view of larger than 180 degrees may be used. In this case, it is possible to secure a field of view of 270 degrees or more as the imaging system 41 of the stereo camera.

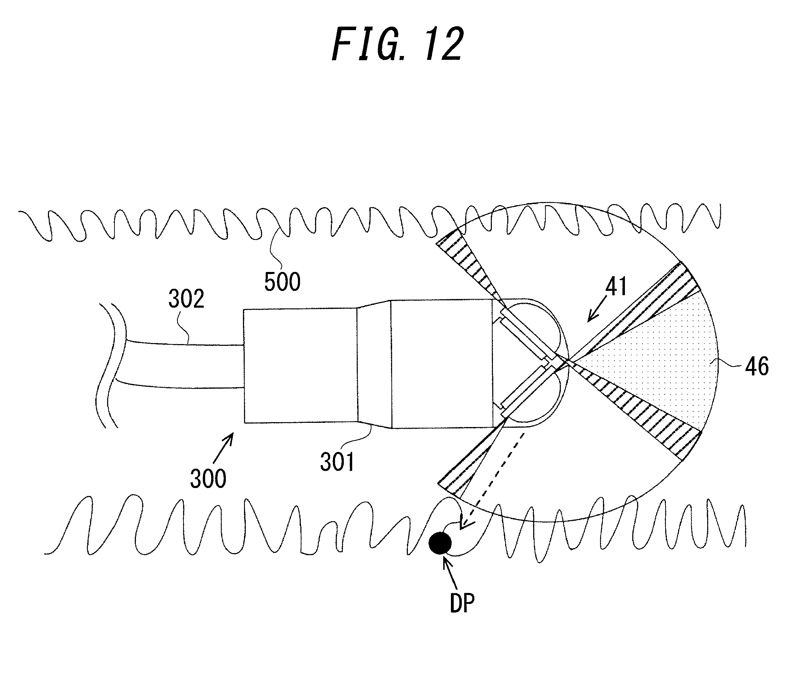

Fig. 12 illustrates an embodiment where the imaging system 41 of the stereo camera according to the embodiment is incorporated into an endoscope 300. In the embodiment, the imaging system 41 of the stereo camera is mounted on a camera unit 301 at the distal end portion of the endoscope 300. Image information of an angle of view of 270 degrees acquired by the imaging system 41 of the stereo camera is recorded in a memory (not illustrated) outside a patient or displayed on a display (not illustrated) outside the patient through a cable 302.

In the embodiment, as illustrated in Fig. 12, since it is possible to allow the field of view of eh endoscope 300 to be significantly a wide angle (270 degrees), it is possible to more reliably detect abnormality of, for example, an affectedportion DP or the like in the figure, that is, the portions which are highly likely to be missed in the related art. In addition, since the distance measurement of the distance to the subject (obstacle) in the area 46 can be performed, it is possible to more reliably prevent an accident of collision of the endoscope 300 to the wall surface of an internal organ 500 to damage the internal organ. In addition, it is possible to perform measurement of a position and size of a lesion.

In addition, in the related art, in order to increase the inspection area of the endoscope, in some cases, the distal end of the endoscope is configured to be bendable so that the direction of the distal end of the endoscope can be controlled. However, according to the embodiment, since the field of view of the endoscope 300 is sufficiently wide, the mechanism of curving the distal end of the endoscope may be omitted. As a result, it is possible to simplify the device and the operation method. In addition, in the imaging system 41 of the stereo camera, since the curved imaging elements are used as described in Figs. 11 (a) and 11 (b), it is possible to miniaturize the distal end portion of the endoscope 300.

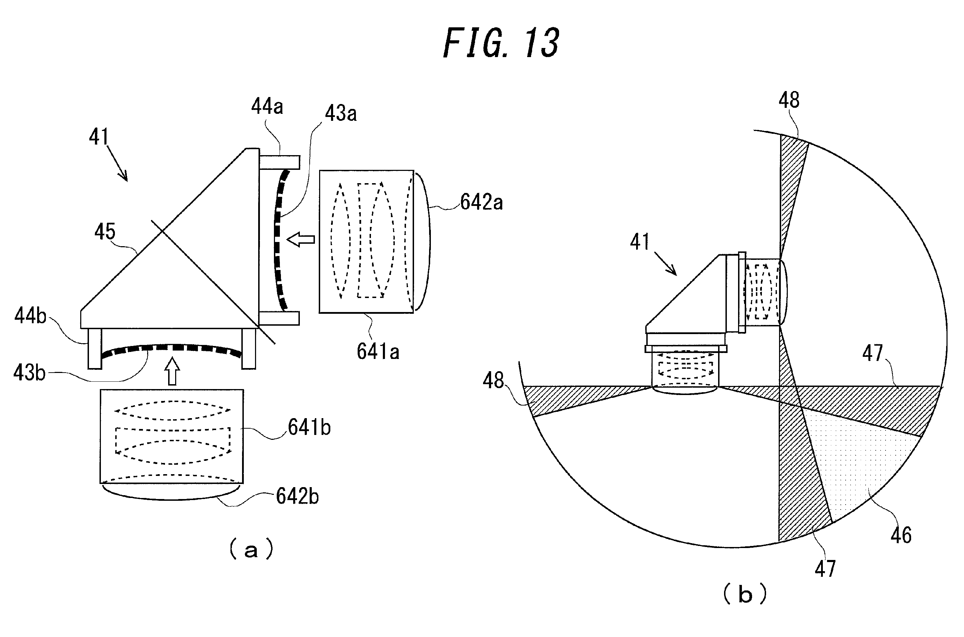

In addition, in the embodiment, the example where a single fisheye lens is used as the left lens system 42a and the right lens system 42b is described. However, a fisheye lens unit is configured with a plurality of lenses, and the fisheye lens unit may be used as the left lens system and the right lens system. Figs. 13 (a) and 13(b) illustrate an example where a plurality of lens groups are incorporated into cases 641a and 641b, a fisheye lens unit having an angle of view of 180 degrees is formed, and the fisheye lens unit is used as a left lens system 642a and a right lens system 642b.

<Sixth Embodiment>

Next, a sixth embodiment of the present invention will be described. In the embodiment, an application example where surveillance of the entire periphery of an object as a measurement reference object and distance measurement are performed by using a plurality of the imaging systems of the stereo camera described in the fifth embodiment will be described.

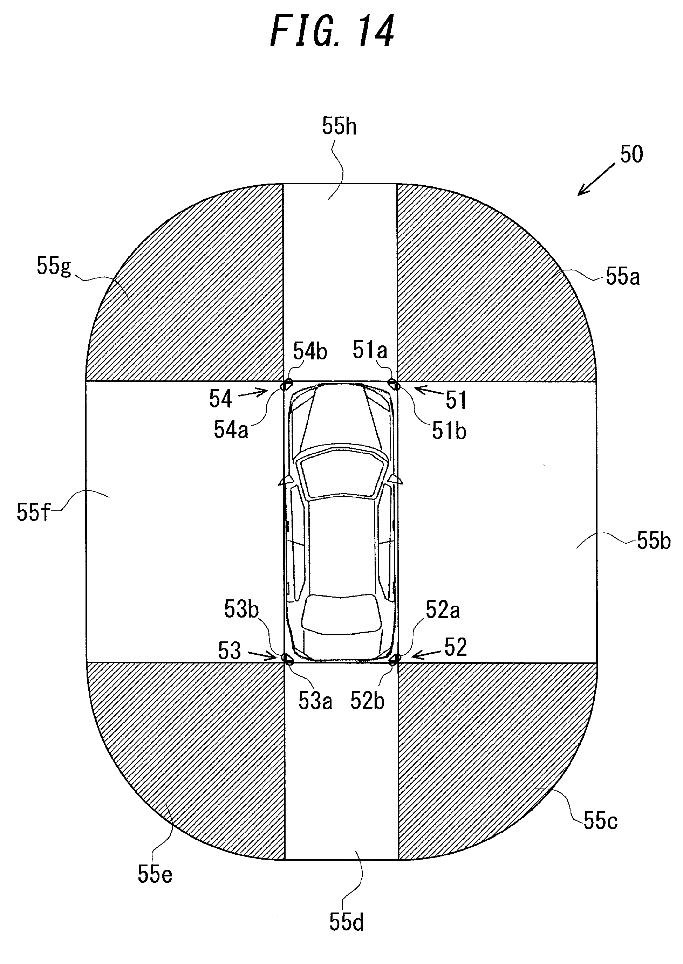

Fig. 14 illustrates a schematic configuration of a stereo camera system 50 where imaging systems 51 to 54 of a stereo camera similar to that described in Figs. 11 (a) and 11(b) are arranged at the four corners of a car. Since the imaging systems 51 to 54 of the stereo camera are arranged in this manner, in the case where surveillance of the periphery of the car in all directions is performed and the car approaches an obstacle, it is possible to perform the distance measurement.

In addition, in the embodiment, particularly with respect to the areas (indicated by hatching) at the four corners of the car, the distance measurement can be performed by using image information obtained by using the four imaging systems. For example, in Fig. 14, with respect to the area 55a, the distance measurement can be performed by using image information obtained by using the four imaging systems 54b, 51a, 51b, and 52a. With respect to the area 55c, the distance measurement can be performed by using image information obtained by using the four imaging systems 51b, 52a, 52b, and 53a. With respect to the area 55e, the distance measurement can be performed by using image information obtained by using the four imaging systems 52b, 53a, 53b, and 54a. With respect to the area 55g, the distance measurement can be performed by using image information obtained by using the four imaging systems 53b, 54a, 54b, and 51a.

Therefore, with respect to the areas 55a, 55c, 55e, and 55g, it is possible to perform the distance measurement based on much more image information, and it is possible to perform the distance measurement by selecting more useful image information, so that it is possible to further increase the accuracy of the distance measurement. In addition, even in the case where the image information is hard to acquire by any one of the imaging systems due to an obstacle, it is possible to continuously perform the distance measurement by using the one of the four image systems which can acquire the image information.

In addition, in Fig. 14, with respect to the area 55b, the distance measurement can be performed by using image information obtained by using the two imaging systems 51b and 52a. With respect to the area 55d, the distance measurement can be performed by using image information obtained by using the two imaging systems 52b and 53a. With respect to the area 55f, the distance measurement can be performed by using image information obtained by using the two imaging systems 53b and 54a. With respect to the area 55h, the distance measurement can be performed by using image information obtained by using the two imaging systems 54b and 51a.

In addition, in the embodiment, in the case of using the imaging systems 51b and 52a, the case of using the imaging systems 52b and 53a, the case of using the imaging systems 53b and 54a, and the case of using the imaging systems 54b and 51a, since the longitudinal and transverse widths of the car may be used as the base line length d, it is possible to secure a very long base line length, and it is possible to improve the accuracy of the distance measurement.

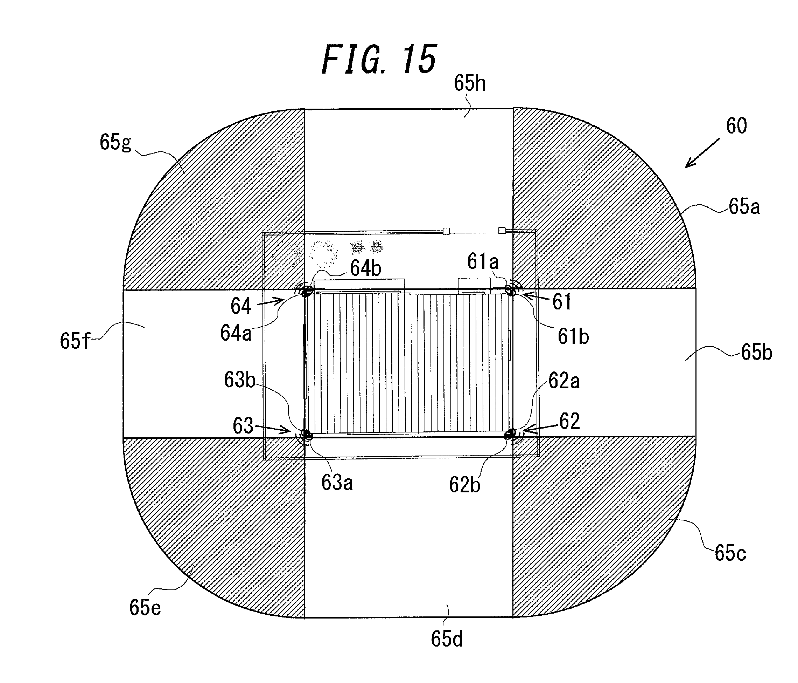

Fig. 15 illustrates a schematic configuration of a stereo camera system 60 where imaging systems 61 to 64 of a stereo camera similar to that described in Figs. 11 (a) and 11(b) are arranged at the four corners of a house. Since the imaging systems 61 to 64 of the stereo camera are arranged in this manner, in the case where surveillance of the periphery of the house in all directions is performed and a suspicious person (suspicious object) approaches, it is possible to perform the distance measurement.

In addition, in the embodiment, particularly with respect to the areas of the four corners, the distance measurement can be performed by using image information obtained by using the four imaging systems. For example, in Fig. 15, with respect to the area 65a, the distance measurement can be performed by using image information obtained by using the four imaging systems 64b, 61a, 61b, and 62a. With respect to the area 65c, the distance measurement can be performed by using image information obtained by using the four imaging systems 61b, 62a, 62b, and 63a. With respect to the area 65e, the distance measurement can be performed by using image information obtained by using the four imaging systems 62b, 63a, 63b, and 64a. With respect to the area 65g, the distance measurement can be performed by using image information obtained by using the four imaging systems 63b, 64a, 64b, and 61a.

Therefore, with respect to the areas 65a, 65c, 65e, and 65g, it is possible to perform the distance measurement based on much more image information, and it is possible to perform the distance measurement by selecting more useful image information, so that it is possible to further increase the accuracy of the distance measurement. In addition, even in the case where the image information is hard to acquire by any one of the imaging systems due to an obstacle, it is possible to continuously perform the distance measurement by using the one of the four image systems which can acquire the image information.

In addition, in Fig. 15, with respect to the area 65b, the distance measurement can be performed by using image information obtained by using the two imaging systems 61b and 62a. With respect to the area 65d, the distance measurement can be performed by using image information obtained by using the two imaging systems 62b and 63a. With respect to the area 65f, the distance measurement can be performed by using image information obtained by using the two imaging systems 63b and 64a. With respect to the area 65h, the distance measurement can be performed by using image information obtained by using the two imaging systems 64b and 61a.

In addition, in the aspect, in the case of using the imaging systems 61b and 62a, the case of using the imaging systems 62b and 63a, the case of using the imaging systems 63b and 64a, and the case of using the imaging systems 64b and 61a, since the longitudinal and transverse widths of the house may be used as the base line length d, it is possible to secure a very long base line length.

<Seventh Embodiment>

Next, a seventh embodiment of the present invention will be described. In the embodiment, another aspect of the application example where surveillance of the entire periphery of an object as a measurement reference object and distance measurement are performed by using a plurality of the imaging systems of the stereo camera described in the fifth embodiment and Figs. 11 (a) and 11(b) will be described.

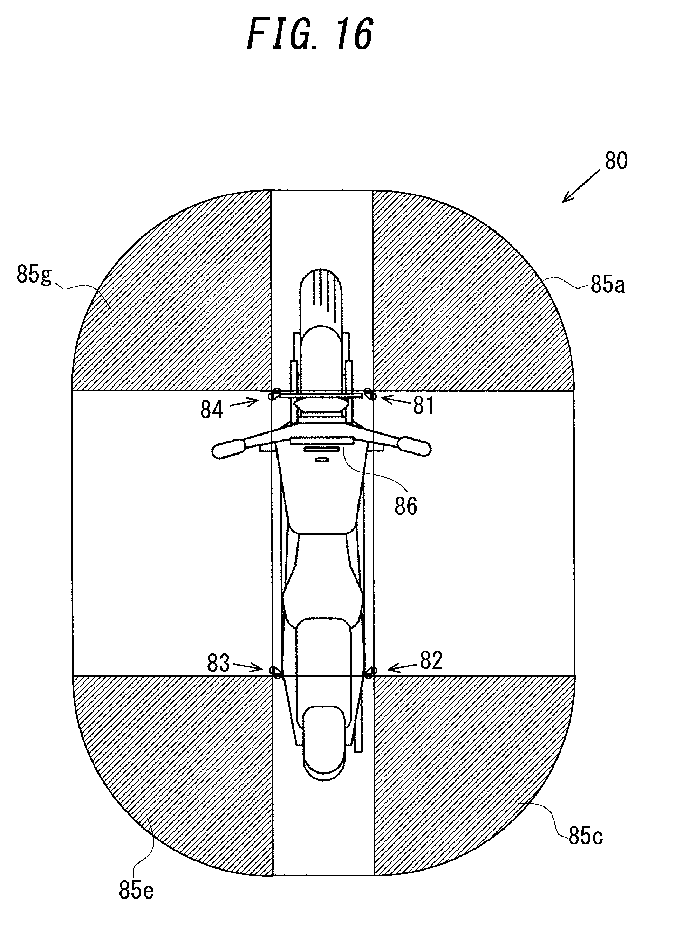

Fig. 16 illustrates a schematic configuration of a stereo camera system 80 where imaging systems 81 to 84 of a stereo camera similar to that described in Figs. 11(a) and 11(b) are arranged at the four corner of a motorcycle. Since the imaging systems 81 to 84 of the stereo camera are arranged in this manner, in the case where surveillance of the periphery of the motorcycle in all directions is performed and the motorcycle approaches an obstacle, it is possible to perform the distance measurement.

In the embodiment, particularly with respect to the areas (indicated by hatching) at the four corners of the motorcycle, the distance measurement can be performed by using image information obtained by using the four imaging systems. Therefore, with respect to the areas 85a, 85c, 85e, and 85g, it is possible to perform the distance measurement based on much more image information, and it is possible to perform the distance measurement by selecting more useful image information, so that it is possible to further increase the accuracy of the distance measurement. In addition, even in the case where there is an obstacle when a measurement object (subject) is imaged by any one of the four imaging systems, it is possible to perform the distance measurement by selecting the imaging system which can image the measurement object (subject).

In addition, as illustrated in Figs. 11(a) and 11(b), in the present invention, since the curved imaging elements are used so as to correct the aberration of the lens systems, it is possible to miniaturize the imaging systems 81 to 84 of the stereo camera. Therefore, the imaging systems 81 to 84 of the stereo camera can be mounted on an object such as a motorcycle. In addition, in the present invention, since the curved imaging elements are used so as to correct the aberration of the lens systems, it is possible to improve the accuracy of the distance measurement. Therefore, the distance measurement can be performed with a short base line length, and even in the case where the width of a motorcycle is selected as a base line length, it is possible to perform accurate distance measurement.

In addition, in the case where the present invention is applied to the motorcycle, for example, the imaging systems 81 and 84 of the stereo camera are mounted on a handle of the motorcycle or a member of integrally rotating with the handle, so that it is possible to the distance measurement in the forward area by selecting the direction where the front wheel of the motorcycle is directed always as the center.

In addition, in the example of Fig. 16, an image monitor 86 is installed, so that it is possible to display distance information and images of the periphery acquired by the stereo camera system 80.

<Eighth Embodiment>

Next, an eighth embodiment of the present invention will be described. In the embodiment, still another aspect of the application example where surveillance of the entire periphery of an object as a measurement reference object and distance measurement are performed by using a plurality of the imaging systems of the stereo camera described in the fifth embodiment and Figs. 11 (a) and 11(b) will be described.

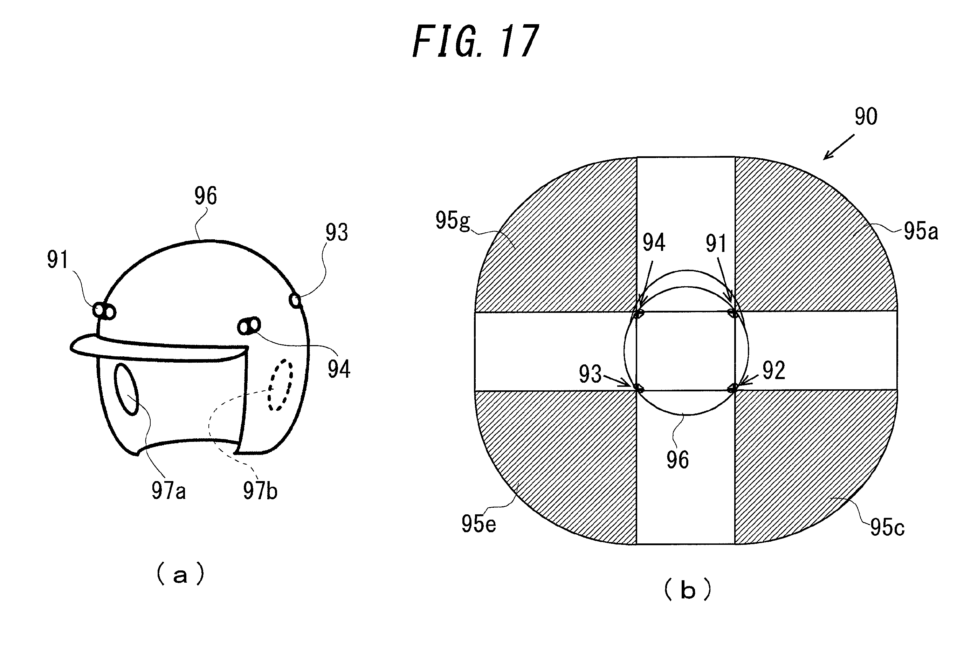

Figs. 17(a) and 17(b) illustrate a schematic configuration of a stereo camera system 90 where imaging systems 91 to 94 of a stereo camera similar to that described in Figs. 11 (a) and 11 (b) are arranged in the periphery of a helmet. Fig. 17(a) illustrates a perspective diagram of a helmet 96 in this case, and Fig. 17 (b) illustrates a plan diagram of the helmet 96 and measurable areas. Since the imaging systems 91 to 94 of the stereo camera are arranged in the periphery of the helmet 96 in this manner, in the case where surveillance of the periphery of a person wearing the helmet 96 in all directions is performed and the person approaches an obstacle, it is possible to perform the distance measurement.

In the embodiment, particularly with respect to the areas (indicated by hatching) at the four corners of the helmet 96, the distance measurement can be performed by using image information obtained by using the four imaging systems. Therefore, with respect to the areas 95a, 95c, 95e, and 95g, it is possible to perform the distance measurement based on much more image information, and it is possible to perform the distance measurement by selecting more useful image information, so that it is possible to further increase the accuracy of the distance measurement. In addition, even in the case where there is an obstacle when a measurement object (subject) is imaged by any one of the four imaging systems, it is possible to perform the distance measurement by selecting the imaging system which can image the measurement object (subject).

In addition, as illustrated in Figs. 11(a) and 11(b), in the present invention, since the curved imaging elements are used so as to correct the aberration of the lens systems, it is possible to miniaturize the imaging systems 91 to 94 of the stereo camera, and it is possible to improve the accuracy of the distance measurement. Therefore, the present invention can be applied to the helmet 96.

In addition, in the embodiment, as illustrated in Fig. 17 (a), speakers 97a and 97b can be mounted inside the helmet 96, so that it is possible to notify the direction or distance of the obstacle to the person wearing the helmet 96 by using warning sound and voice. In addition, the helmet 96 may be a helmet which is worn when a person boards a transportation device such a car, a motorcycle, or an airplane or a helmet used in construction sites or the like. In addition, the helmet may be a helmet used in climbing or during walking on the street. The function as a helmet may be used according to the purpose. In addition, in the case where the helmet is used during walking on the street, the image information acquired by the imaging systems 91 to 94 of the stereo camera may be allowed to be viewed by using a smart phone, a mobile phone, or a tablet terminal (not illustrated).

<Ninth Embodiment>

Next, a ninth embodiment of the present invention will be described. In the embodiment, still another aspect of the application example where surveillance of the entire periphery of an object as a measurement reference object and distance measurement are performed by using a plurality of the imaging systems of the stereo camera described in the fifth embodiment and Figs. 11 (a) and 11(b) will be described.

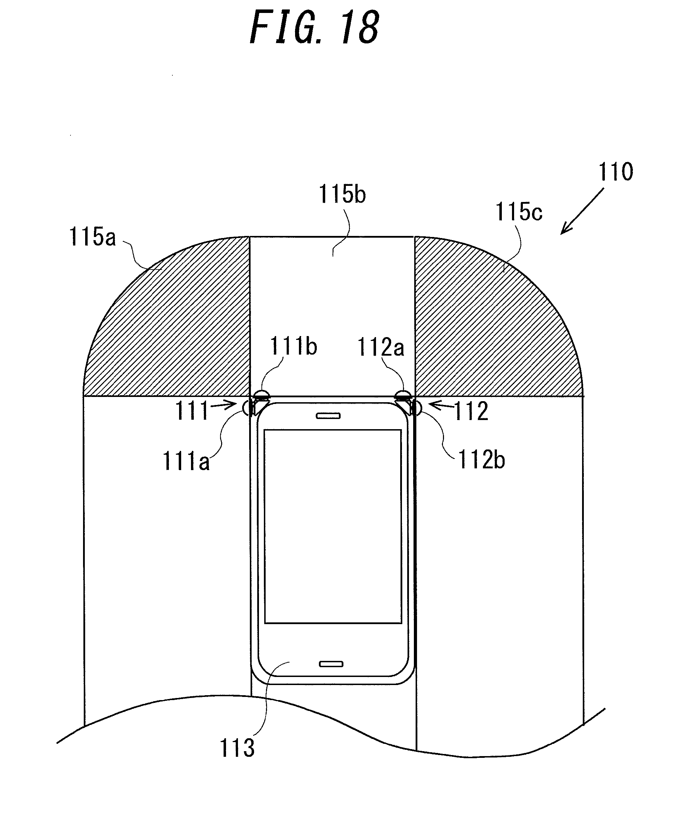

Fig. 18 illustrates a schematic configuration of a stereo camera system 110 where imaging systems 111 and 112 of a stereo camera similar to that described in Figs. 11 (a) and 11 (b) are arranged at both sides of the upper end of a smart phone 113. Since the imaging systems 111 and 112 of the stereo camera are arranged in the periphery of the smart phone 113 in this manner, in the case where surveillance of the wide range of a forward area and a sideward area of a user of the smart phone 113 is performed and the user approaches an obstacle, it is possible to perform the distance measurement.

In the embodiment, particularly with respect to the areas (indicated by hatching) in the forward-side inclined directions, the distance measurement can be performed by using image information obtained by the three imaging systems. For example, in Fig. 18, with respect to an area 115a, the distance measurement can be performed by using image information obtained by using the three imaging systems 111a, 111b, and 112a. With respect to an area 115c, the distance measurement can be performed by using image information obtained by using the three imaging systems 111b, 112a, and 112b. Therefore, with respect to the areas 115a and 115c, it is possible to perform the distance measurement based on much more image information, and it is possible to perform the distance measurement by selecting more useful image information, so that it is possible to further increase the accuracy of the distance measurement. In addition, even in the case where there is an obstacle when a measurement object (subject) is imaged by any one of the three imaging systems, it is possible to perform the distance measurement by selecting the imaging system which can image the measurement object (subject).

In addition, as illustrated in Figs. 11(a) and 11(b), in the present invention, since the curved imaging elements are used so as to correct the aberration of the lens systems, it is possible to miniaturize the imaging systems 111 to 112 of the stereo camera, and it is possible to improve the accuracy of the distance measurement. Therefore, the present invention can be applied to the smart phone 113.

In the embodiment, through a combination of applications of the smart phone 113 and the imaging systems 111 and 112 of the stereo camera, it is possible to implement various usage aspects. For example, by adding image information and distance information obtained by the imaging systems 111 and 112 of the stereo camera to GPS information or information from a gyro sensor, it is possible to improve the accuracy of a navigation system for street walking.

In addition, for example, by displaying the image information by the imaging systems 111 and 112 of the stereo camera on a portion of the display of the smart phone 113, the image including a wide range from the feet to the head of the user can be displayed, and thus, the user can safely take a walk while using the smart phone 113. In addition, when the distance to an obstacle is a predetermined distance or less, an alert display is performed, so that the user can more safely take a walk.

In addition, the smart phone 113 may be used as a distance measurement device without a change, or may be used as an ultra-wide-angle digital camera.

<Tenth Embodiment>

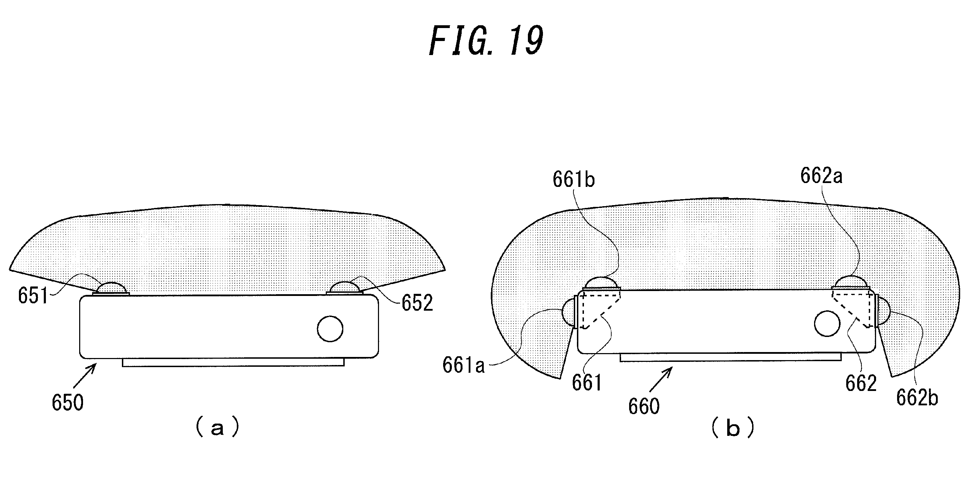

Next, a tenth embodiment of the present invention will be described. In the embodiment, an example where the two imaging systems of the stereo camera described in the fifth embodiment and Figs. 11(a) and 11(b) are incorporated into a 3D camera (hereinafter, the camera is also referred to as a 3D sphere camera in terms of a function of enabling a spherical viewing angle) will be described. Figs. 19(a) and 19(b) illustrate plan diagrams and fields of views of a 3D camera 650 where a pair of fisheye lenses are incorporated as left and right lens systems of the 3D camera and a 3D sphere camera 660 where the two imaging systems of the stereo camera described in the fifth embodiment and Figs. 11 (a) and 11 (b) are incorporated as left and right imaging systems.

Fig. 19(a) illustrates the plan diagram and the field of view of the 3D camera 650 configured to include a left imaging system 651 and a right imaging system 652. As the lens systems of the left imaging system 651 and the right imaging system 652, fisheye lenses having an angle of view of about 180 degrees are used. In addition, although not illustrated, as the imaging elements of the left imaging system 651 and the right imaging system 652, curved imaging elements are used in order to correct the optical aberration of the peripheral portions of the fisheye lenses. In the 3D camera 650 illustrated in Fig. 19(a), since the image distortion in the peripheral portions of the imaging elements can be corrected, it is possible to obtain a high-quality 3D image. On the other hand, in the 3D camera 650, the field of view of the combination of the left imaging system 651 and the right imaging system 652 is also about 180 degrees and does not exceed the field of view (angle of view) of the fisheye lens of the related art.

On the other hand, in the 3D sphere camera 660 illustrated in Fig. 19(b), it is possible to obtain a field of view which is totally close to 360 degrees. In addition, as a total of the four imaging elements used for the 3D sphere camera 660, curved image elements are used in order to correct the optical aberration in the peripheral portions of all the corresponding fisheye lenses. Therefore, it is possible to obtain a high-quality 3D image in the field of view which is totally close to 360 degrees. In addition, accordingly, it is possible to perform the image representation at the viewing angle of a human.

<Eleventh Embodiment>

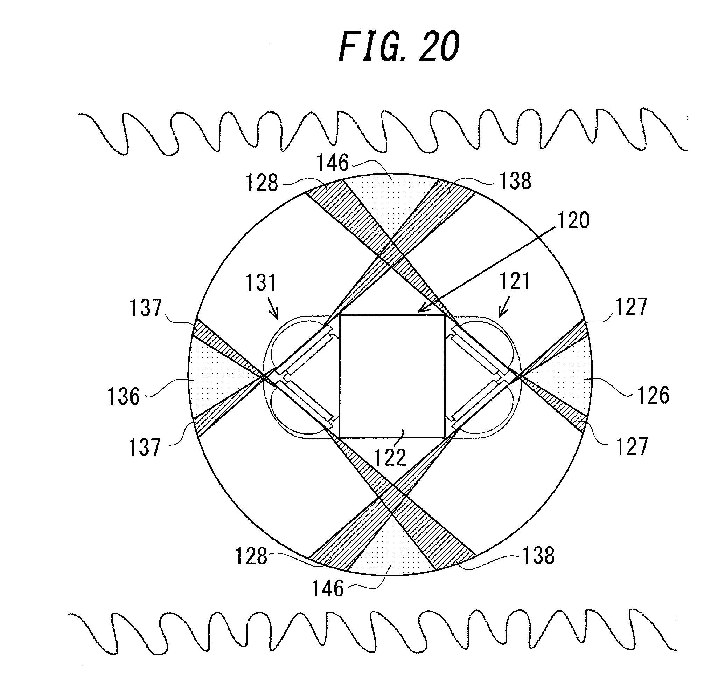

Fig. 20 illustrates a schematic configuration of a capsule-type endoscope system 120 where imaging systems 121 and 131 of a stereo camera similar to that described in Figs. 11 (a) and 11(b) are arranged at both ends of a camera unit 122. Since the fields of view of the imaging systems 121 and 131 of the stereo camera are 270 degrees, it is possible to obtain image information of the periphery of the endoscope system 120 in all directions. In addition, with respect to areas 126 (including an area 127), 136 (including an area 137), and 146 (including areas 128 and 138) where the angles of view of the lens systems overlap, the distance measurement can be performed. Therefore, in the embodiment, since curved imaging elements are used in order to correct the aberration of the lens systems of the imaging systems, it is possible to miniaturize the imaging systems 121 and 131 of the stereo camera, and it is possible to improve the accuracy of the distance measurement in the areas 127, 128, 137, and 138.

The image information of the angle of view of 270 degrees acquired by the imaging system 121 and 131 of the stereo camera is transmitted through communication to a reception device (not illustrated) arranged outside the body of a patient, recorded in a memory, and displayed on a display. Otherwise, the image information is stored in a memory inside the camera unit 122. In the embodiment, since the image information of the peripheral portion of the endoscope system 120 in all directions can be acquired, it is possible to improve the accuracy of the detection of abnormal portions by the capsule-type endoscope system 120.

<Twelfth Embodiment>

Next, a twelfth embodiment of the present invention will be described. In the embodiment, an aspect where the imaging systems of the stereo camera illustrated in the fifth embodiment and Figs. 11 (a) and 11(b) are arranged in front and rear at an object as a measurement reference object and surveillance and distance measurement in the substantially entire periphery of the object are performed will be described.

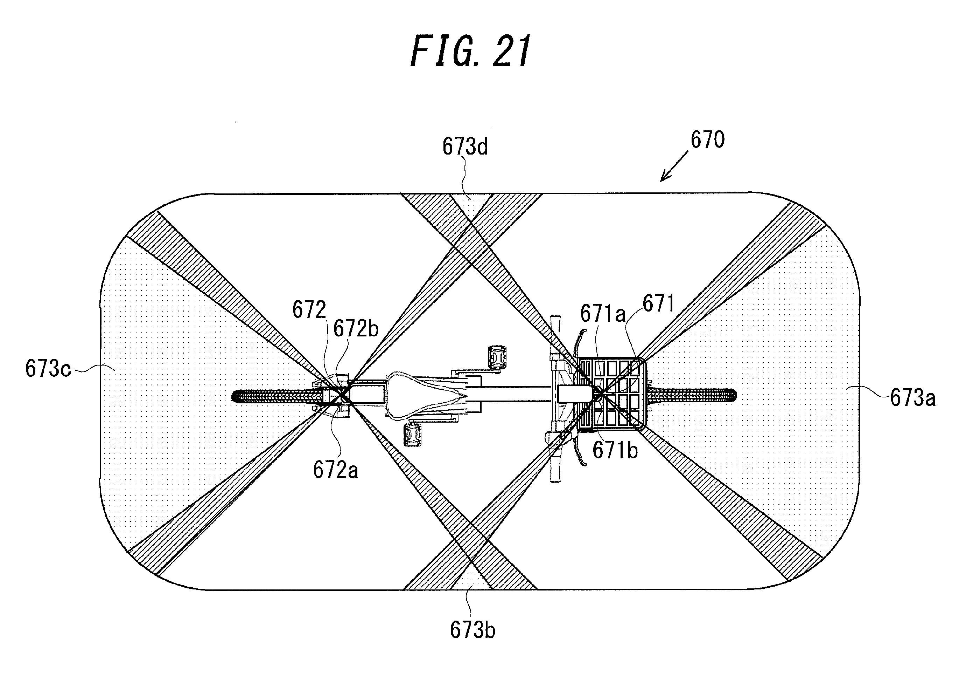

Fig. 21 illustrates a schematic configuration of a stereo camera system 670 where imaging systems 671 and 672 of a stereo camera similar to that described in Figs. 11 (a) and 11 (b) are arranged in front and rear at a bicycle. In the embodiment, since the bicycle having a narrow width is selected as the object, the imaging system 671 of the stereo camera is arranged in the forward area of the bicycle and the imaging system 672 is arranged in the backward area of the bicycle. Therefore, in the case where surveillance of the forward and backward directions and the leftward and rightward directions of the object in all directions performed and the bicycle approaches an obstacle, it is possible to perform the distance measurement.

Like the embodiment, in the object having a narrow width such as a bicycle, with respect to the forward area 673a, by using image information obtained by a left imaging system 671a and a right imaging system 671b installed in the imaging system 671 arranged in the forward area, it is possible to perform the distance measurement stably with a high accuracy. Similarly with respect to the backward area 673c, by using image information obtained by a left imaging system 672a and a right imaging system 672b installed in the imaging system 672, it is possible to perform the distance measurement stably with a high accuracy.

In addition, with respect to the area 673b in the rightward direction with respect to the forward direction of the bicycle, since there is no part or the like particularly interfering with the measurement, it is possible to perform the distance measurement by using the image information obtained by the right imaging system 671b of the imaging system 671 and the left imaging system 672a of the imaging system 672. Similarly, with respect to the area 673d in the leftward direction with respect to the forward direction of the bicycle, it is possible to perform the distance measurement by using the image information obtained by the left imaging system 671a of the imaging system 671 and the right imaging system 672b of the imaging system 672. In addition, in Fig. 21, although the area 673b and the area 673d appear to be narrow areas, the measureable range is increased as the distance from the bicycle is increased, so that it is possible to measure sufficient areas.

In this manner, in the embodiment, the stereo cameras are arranged in front and rear at the object having a narrow width such as a bicycle, and the distance measurement of the areas of the object in the forward, backward, leftward, and rightward directions is performed based on the information obtained by the left and right imaging systems of the stereo cameras. Therefore, it is possible to perform the distance measurement of sufficiently large areas in the periphery of the object without an increase in cost by using a smaller number of the stereo cameras.

<Thirteenth Embodiment>

Next, a thirteenth embodiment of the present invention will be described. In the embodiment, an application example where surveillance and distance measurement of the periphery of an object as a measurement reference object in all directions are performed by using a plurality of imaging systems of a stereo camera and the imaging system used for the distance measurement is switched according to the distance to a subject (measurement object) will be described.

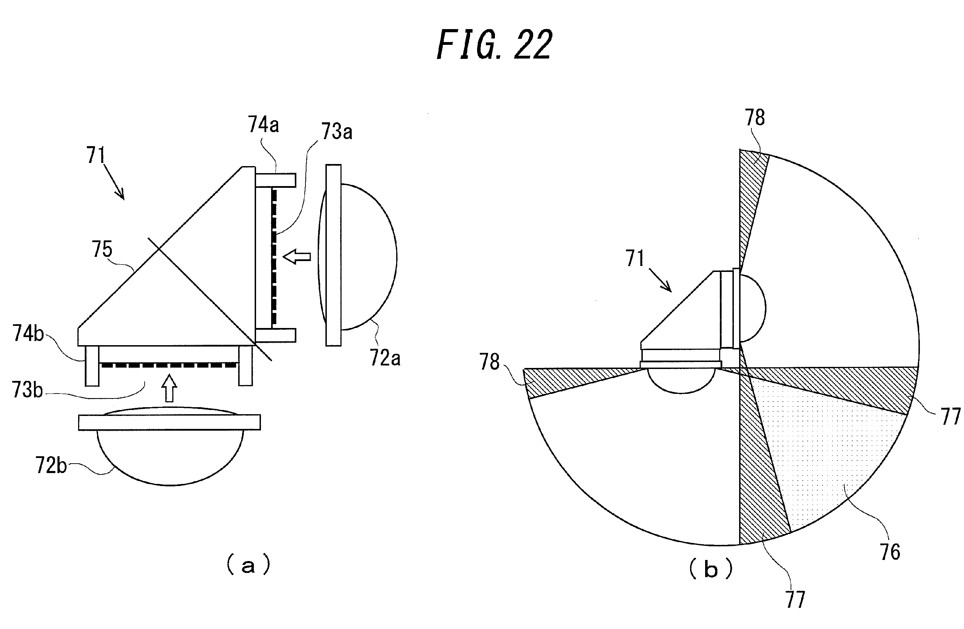

Figs. 22(a) and 22(b) illustrate a schematic configuration of an imaging system 71 of a stereo camera according to the embodiment. Fig. 22 (a) is a schematic configuration diagram of the case where the imaging system 71 of the stereo camera is viewed from the upper portion, and Fig. 22 (b) is a diagram for explaining usage states.

The configuration of the imaging system 71 of the stereo camera according to the embodiment is equivalent to the configuration of the imaging system 41 of the stereo camera described in Figs. 11 (a) and 11 (b) except that a left imaging element 73a and a right imaging element 73b are formed with planar surfaces. In the embodiment, the imaging system 71 of the stereo camera is applied to the stereo camera systems 50 and 60 described in Figs. 14 and 15. Hereinafter, in the embodiment, although the description is made with reference to Figs. 14 and 15, it is assumed that the same imaging systems as the imaging system 71 of the stereo camera are used as imaging systems 51 to 54 and 61 to 64 of the stereo camera.

In Fig. 14, at the start time of the distance measurement of the periphery of a car, the distance measurement is performed by using all the imaging system 51 to 54 of the stereo camera. Therefore, for example, in the case where a subject (measurement object) exists in a somewhat far distance in the forward area of the car, the distance measurement is performed by using the imaging systems 54b and 51a. After that, in the case where the subject (measurement object) approaches the car to be in the area 55g and the distance becomes a predetermined value or less, the distance measurement is switched to the measurement using the imaging systems 54a and 54b. Similarly, in the case where the subject (measurement object) is in the area 55a and the distance becomes a predetermined value or less, the distance measurement is switched to the measurement using the imaging systems 51a and 51b.

Herein, although the base line length d is short in the measurement using the imaging systems 54a and 54b or the measurement using the imaging systems 51a and 51b, since the position accuracy between the imaging systems is high, it is possible to perform the more-accurate distance measurement.

According to the embodiment, in the case where the subject (measurement object) exists in a relatively far distance from the car, the distance measurement of the periphery of the car in all directions is performed, and in the stage where the subject (measurement object) is close to the car, the distance measurement can be switched to the distance measurement having a higher accuracy. Therefore, it is possible to more accurately detect an obstacle which exists in a relatively far distance, and when the obstacle is close thereto, the more-accurate distance measurement is performed, so that it is possible to contribute to collision avoidance.

In addition, in the embodiment, although the case where the subject (measurement object) exists in the backward area of the car in the right transverse direction and the left transverse direction is omitted in description, similar distance measurement may be performed.

Next, in Fig. 15, at the start time of the distance measurement of the periphery of a house, the distance measurement is performed by using all the imaging systems 61 to 64 of the stereo camera. Therefore, for example, in the case where a subject (measurement object) exists in a somewhat far distance in front of the house, the distance measurement is performed by using the imaging systems 64b and 61a. After that, in the case where the subject (measurement object) approaches the house to be in the area 65g and the distance becomes a predetermined value or less, the distance measurement is switched to the measurement using the imaging systems 64a and 64b. Similarly, in the case where the subject (measurement object) is in the area 65a and the distance becomes a predetermined value or less, the distance measurement is switched to the measurement using the imaging systems 61a and 61b.

In this aspect, in the case where a suspicious person exists in a relatively far distance from the house, the distance measurement of the periphery of the house in all directions is performed, and in the stage where the suspicious person is close to the house, the distance measurement can be switched to the distance measurement having a higher accuracy. Therefore, it is possible to more accurately detect a suspicious person who exists in a relatively far distance, and when the suspicious person is close to the house, the more-accurate distance measurement is performed, so that it is possible to contribute to prevention of intrusion of the suspicious person.

In addition, in the aspect, although the case where the suspicious person exists in the rear side of the house in the right transverse direction and the left transverse direction is omitted in description, similar distance measurement may be performed.

In addition, as described above in the embodiment, the description is made under the assumption that the imaging system 71 of the stereo camera illustrated in Figs. 22(a) and 22(b) is used. This means that the imaging system of the stereo camera according to the embodiment is not limited to the imaging system using the image element formed with the curved surface as illustrated in Figs. 11(a) and 11(b), and the use of the imaging system 41 of the stereo camera as the imaging system of the stereo camera according to the embodiment is not excluded. In addition, any imaging system of a stereo camera having different configurations may be used as long as the imaging system obtains the functions and effects similar to the above-described functions and effects.

In addition, in the embodiment, although the example where the fisheye lens having an angle of view of 180 degrees is used as the imaging system of the stereo camera is described, the angle of view of the lens of the imaging system is not limited to 180 degrees. If the imaging systems have an angle of view of 135 degrees or more, it is possible to secure a field of view of 270 degrees as the imaging systems, and it is possible to secure the portion (distance measureable portion) where the angles of view overlap each other. In addition, a fisheye lens having an angle of view of 180 degrees or more may be used.

In addition, in all the above-described embodiments, although the example where the two imaging system of the left and right imaging systems are included in a single stereo camera is described, the number of imaging systems is not limited to 2. The present invention can be applied to a stereo camera which performs measurement of the distance to a subject or generation of a stereoscopic image by using three imaging systems or more.

REFERENCE SIGNS LIST

1, 11, 21 Imaging system of stereo camera
1a, 11a Left imaging system
1b, 11b Right imaging system
2a, 12a, 22a, 42a, 72a Left lens system
2b, 12b, 22b, 42b, 72b Right lens system
3a, 13a, 23a, 43a, 73a Left imaging element
3b, 13b, 23b, 43b, 73b Right imaging element
10, 30 Stereo camera
41, 51 ~ 54, 61 ~ 64, 71 Imaging system of stereo camera
50, 60, 80, 90, 110, 120, 670 Stereo camera system
81 ~ 84, 91 ~ 94 Imaging system of stereo camera
111, 112, 121, 131 Imaging system of stereo camera
661, 662, 671, 672 Imaging system of stereo camera

**Claims**

1. A stereo camera which includes a plurality of imaging units, each of which is configured to include an optical system and an imaging element on which light passing through the optical system is focused to derive a distance to a predetermined subject or to generate a stereoscopic image containing the subject based on an image signal acquired by imaging the subject by the imaging units **characterized in that**; a light receiving surface of the imaging element in at least a portion of the imaging units is curved in an optical axis direction so as to correct aberration of an image plane of the optical system which forms an image of the subject on the imaging element.

2. The stereo camera according to claim 1, wherein the imaging units are arranged so that optical axes intersect each other with a predetermined angle of convergence, and wherein instead of changing the angle of convergence according to the subject, an area for acquisition of the image signal in the light receiving surface of the imaging element is changed according to the subject.

3. The stereo camera according to claim 1 or 2, wherein at least a portion of the optical systems in the imaging units are integrally formed.

4. The stereo camera according to claim 3, wherein the imaging elements on which light passing through the integrally-formed optical systems is focused are integrally formed.

5. A command signal discrimination device which discriminates a command signal input to a predetermined apparatus based on the image signal acquired in the stereo camera according to any one of claims 1 to 4.

6. The stereo camera according to claim 1, further comprising:

a holding unit which holds the imaging units so that optical axes thereof do not intersect each other in front of the optical axes and angles of view of the optical systems of the imaging units overlap each other; and
a distance measuring unit which detects the distance to the subject from the image signal of the subject imaged by the imaging units in the portion where the angles of view overlap.

7. The stereo camera according to claim 6, wherein each angle of view of the optical systems is larger than 135 degrees.

8. The stereo camera according to claim 7, wherein the holding unit holds the two imaging units so that an angle between the optical axes is 90 degrees.

9. The stereo camera according to claim 6 or 7, wherein the holding unit allows a size of an overlap portion of the angles of view to be changeable by changing a holding angle of the optical systems.

10. A stereo camera system which includes a plurality of the stereo cameras according to any one of claims 6 to 9 to acquire an image signal by imaging a predetermined subject by at least one of the stereo cameras and to derive a distance from a measurement reference object to the subject based on the image signal,
wherein the stereo cameras are arranged directly to the measurement reference object so as to image an outside from the measurement reference object, and
wherein the distance from the measurement reference object to the subject is derived by using the image signal acquired by two or more of the stereo cameras.

11. The stereo camera system according to claim 10, wherein the measurement reference object is a car, and the stereo cameras are arranged at four corners of the car.

12. The stereo camera system according to claim 10, wherein the measurement reference object is a house, and the stereo cameras are arranged at convex-shaped corners of the house.

13. The stereo camera system according to any one of claims 10 to 12, wherein the stereo cameras are arranged to the measurement reference object so that the distance measurement of a periphery of the measurement reference object in all directions can be performed by the stereo cameras.

14. The stereo camera system according to any one of claims 10 to 13, wherein in the case where the distance between the measurement reference object and the subject is the predetermined distance or less, the distance from the measurement reference object to the subject is derived by using one of the stereo cameras.

15. A stereo camera system which includes a plurality of the stereo cameras to acquire an image signal by imaging a predetermined subject by at least one of the stereo cameras and to derive a distance from a measurement reference object to the subject based on the image signal,
wherein the stereo cameras are arranged directly to the measurement reference object so as to image an outside from the measurement reference object, and
wherein the distance from the measurement reference object to the subject is derived by using the image signal acquired by two or more of the stereo cameras.

16. The stereo camera system according to claim 15, wherein the measurement reference object is a car, and the stereo cameras are arranged at four corners of the car.

17. The stereo camera system according to claim 15, wherein the measurement reference object is a house, and the stereo cameras are arranged at convex-shaped corners of the house.

18. The stereo camera system according to any one of claims 15 to 17, wherein the stereo cameras are arranged to the measurement reference object so that the distance measurement of the entire periphery of the measurement reference object can be performed by the stereo cameras.

19. The stereo camera system according to any one of claims 15 to 18, wherein in the case where the distance between the measurement reference object and the subject is the predetermined distance or less, the distance from the measurement reference object to the subject is derived by using one of the stereo cameras.

20. The stereo camera system according to any one of claims 15 to 19,
wherein the stereo camera includes two imaging units, each of which includes an optical system having an angle of view of 135 degrees or more and an imaging element on which light passing through the optical system is focused, and
wherein the stereo camera further includes a holding unit which holds the two imaging units so that an angle between the optical axes is 90 degrees.

# FIG. 1

# FIG. 2

FIG. 3

# FIG. 4

# FIG. 5

(b)

(a)

# FIG. 6

# FIG. 7

# FIG. 8

# FIG. 9

# FIG. 10

# FIG. 11

( a )

( b )

*FIG. 12*

FIG. 13

# FIG. 14

# FIG. 15

EP 2 846 531 A1

# FIG. 16

# FIG. 17

（a）

（b）

EP 2 846 531 A1

# FIG. 18

FIG. 19

(a)

(b)

FIG. 20

FIG. 21

EP 2 846 531 A1

# FIG. 22

( a )

( b )

EP 2 846 531 A1

## INTERNATIONAL SEARCH REPORT

| International application No. |
| --- |
| PCT/JP2013/062672 |

### A. CLASSIFICATION OF SUBJECT MATTER
*H04N5/225*(2006.01)i, *G02B7/28*(2006.01)i, *G02B7/30*(2006.01)i, *G03B35/20* (2006.01)i, *G03B37/00*(2006.01)i, *H04N5/369*(2011.01)i, *H04N13/02*(2006.01)i

According to International Patent Classification (IPC) or to both national classification and IPC

### B. FIELDS SEARCHED

Minimum documentation searched (classification system followed by classification symbols)
H04N5/225, G02B7/28, G02B7/30, G03B35/20, G03B37/00, H04N5/369, H04N13/02

Documentation searched other than minimum documentation to the extent that such documents are included in the fields searched
Jitsuyo Shinan Koho        1922–1996   Jitsuyo Shinan Toroku Koho   1996–2013
Kokai Jitsuyo Shinan Koho   1971–2013   Toroku Jitsuyo Shinan Koho    1994–2013

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| Y | JP 2012-32964 A  (Olympus Imaging Corp.), 16 February 2012 (16.02.2012), paragraphs [0016] to [0023], [0049] to [0050]; fig. 1 & US 2012/0019528 A1 | 1-14 |
| Y | JP 2004-356175 A  (Matsushita Electric Industrial Co., Ltd.), 16 December 2004 (16.12.2004), paragraph [0025] (Family: none) | 1-14 |
| Y | JP 2008-286527 A  (Panasonic Corp.), 27 November 2008 (27.11.2008), paragraphs [0022], [0041] to [0043]; fig. 1, 3 (Family: none) | 3,4 |

| ☒ Further documents are listed in the continuation of Box C. | ☐ See patent family annex. |
| --- | --- |

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 20 May, 2013 (20.05.13) | 28 May, 2013 (28.05.13) |

| Name and mailing address of the ISA/ Japanese Patent Office | Authorized officer |
| --- | --- |
| Facsimile No. | Telephone No. |

Form PCT/ISA/210 (second sheet) (July 2009)

**INTERNATIONAL SEARCH REPORT**

International application No.

PCT/JP2013/062672

C (Continuation).  DOCUMENTS CONSIDERED TO BE RELEVANT

| Category* | Citation of document, with indication, where appropriate, of the relevant passages | Relevant to claim No. |
|---|---|---|
| Y | JP 2012-15660 A  (Fujifilm Corp.),<br>19 January 2012 (19.01.2012),<br>paragraphs [0021] to [0025]<br>(Family: none) | 5 |
| Y | JP 2001-312018 A  (Kabushiki Kaisha Riaruai),<br>09 November 2001 (09.11.2001),<br>paragraphs [0041] to [0043]; fig. 4<br>(Family: none) | 6-14 |
| Y | JP 2011-204118 A  (Konica Minolta Opto, Inc.),<br>13 October 2011 (13.10.2011),<br>fig. 13<br>(Family: none) | 10-20 |
| Y | JP 2010-258669 A  (Fujifilm Corp.),<br>11 November 2010 (11.11.2010),<br>paragraphs [0012] to [0017]<br>(Family: none) | 15-20 |

Form PCT/ISA/210 (continuation of second sheet) (July 2009)

**EP 2 846 531 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012053303 A
- JP 2007101662 A
- JP 2005278133 A
- JP 2001284564 A
- JP 7095623 A